(19) Europäisches Patentamt
European Patent Office
Office européen des brevets

(11) Numéro de publication: **0 165 134 B1**

(12) # FASCICULE DE BREVET EUROPEEN

(45) Date de publication de fascicule du brevet: **03.02.93**

(51) Int. Cl.5: **C07H 3/06**, A61K 31/70

(21) Numéro de dépôt: **85400953.7**

(22) Date de dépôt: **15.05.85**

(54) **Nouveaux oligosaccharides, leur préparation par voie de synthèse et leurs applications biologiques.**

(30) Priorité: **16.05.84 FR 8407589**

(43) Date de publication de la demande:
**18.12.85 Bulletin 85/51**

(45) Mention de la délivrance du brevet:
**03.02.93 Bulletin 93/05**

(84) Etats contractants désignés:
**AT BE CH DE GB IT LI LU NL SE**

(56) Documents cités:
**EP-A- 0 027 089**
**EP-A- 0 048 231**
**EP-A- 0 084 999**

**CHEMICAL ABSTRACTS, vol. 101, no. 21, 19 novembre 1984, page 45, ref.no. 183714g, Columbus, Ohio, US; J. FAREED et al.: "Antithrombotic actions and pharmacokinetics of heparin fractions and fragments" & NOUV.REV.FR. HEMATOL. 1984, 26 (4), 267-75**

(73) Titulaire: **CHOAY S.A.**
**40 Avenue George V**
**F-75008 Paris(FR)**

(72) Inventeur: **Petitou, Maurice**
**27, rue du Javelot**
**F-75645 Paris Cedex 13(FR)**
Inventeur: **Lormeau, Jean-Claude**
**1, rue Joseph Delattre**
**F-76150 Maromme(FR)**
Inventeur: **Choay, Jean**
**21, rue Saint-Guillaume**
**F-75007 Paris(FR)**
Inventeur: **Jacquinet, Jean-Claude**
**4, allée Georges Brassens**
**F-45100 Orleans La Source(FR)**
Inventeur: **Sinay, Pierre**
**5, rue Jacques Monod**
**F-45100 Orleans La Source(FR)**

(74) Mandataire: **Polus, Camille et al**
**c/o Cabinet Lavoix 2, Place d'Estienne d'Orves**
**F-75441 Paris Cedex 09(FR)**



Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

L'invention a pour objet de nouveaux oligosaccharides, leur préparation par voie de synthèse et leurs applications biologiques.

Il s'agit d'un développement de l'invention faisant l'objet de la demande de brevet EP No 83 400110 du 17 janvier 1983. Dans cette demande de brevet, on a décrit un procédé d'obtention par synthèse organique d'oligosaccharides possédant la structure de fragments de chaînes de mucopolysaccharides acides naturels, et de dérivés de ces oligosaccharides. On rappelle que l'expression "mucopolysaccharide acide" désigne des dérivés également couramment appelés glycosaminoglycuronoglycanes. Ces dérivés sont formés d'oligosaccharides et de polysaccharides constituant plus spécialement les chaînes de dérivés biologiquement actifs tels que ceux du type héparine ou héparanesulfate. Ces chaînes sont essentiellement formées de motifs alternés sucre aminé-acide uronique, ou inversement. Dans ces motifs, le sucre aminé présente plus spécialement une structure D-glucosamine (ou a) et l'acide uronique une structure acide-D-glucuronique (ou b) ou acide L-iduronique (ou c).

La grande souplesse du procédé décrit dans cette demande de brevet EP permet de préparer l'enchaînement des motifs souhaité, selon la stéréochimie desirée et avec des substitutions déterminées. Il est ainsi possible d'obtenir notamment des oligosaccharides constituant des analogues de structure de fragments de chaînes d'héparine.

Ces fragments peuvent comprendre avantageusement l'enchaînement octasaccharidique ABC-DEFGH,obtenu jusqu'alors par la Demanderesse par dépolymérisation enzymatique de structure I

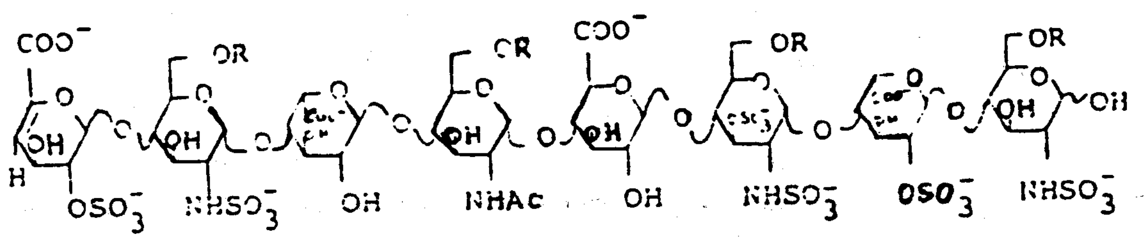

A B C D E F G H

Les oligosaccharides obtenus par voie de synthèse peuvent également comprendre seulement une partie de cet enchaînement où être constitués par cet enchaînement.

Les lettres A à H indiquées sous la formule, désignent, telles qu'utilisées dans la description, un type de structure, les substitutions pouvant être identiques ou différentes de celles de la formule.

Comme décrit dans cette demande de brevet principal, les oligosaccharides élaborés constituent des réactifs biologiques et des substances de référence particulièrement intéressants. Ils sont, de plus, dotés de propriétés pharmacologiques leur conférant une utilité de grande importance en tant que principe actif de médicaments.

Certains de ces oligosaccharides se sont révélés plus spécialement actifs dans le domaine de la coagulation sanguine.

Ainsi, si une activité anti-Xa (mesurée par le titre Yin-Wessler) a pu être décelée chez un trisaccharide de structure DEF, c'est avec un pentasaccharide de structure DEFGH qu'il a été possible de mettre en évidence une affinité très élevée pour l'antithrombine III ou ATIII et une activité anti-Xa (Yin-Wessler) très élevée d'au moins 2000 unités Yin-Wessler/mg.

Ce produit répond à la formule suivante :

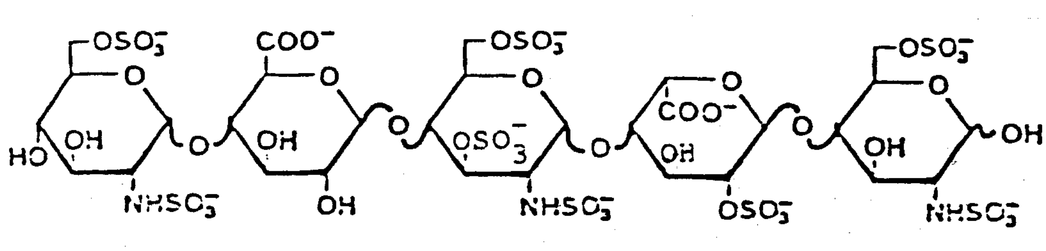

On a pu mettre en évidence une activité anti-Xa d'au moins 2000 unités Yin-Wessler/mg.

En poursuivant leurs travaux, les inventeurs ont à présent constaté d'une manière surprenante, une activité suffisamment élevée pour permettre une exploitation en tant que principe actif de médicament antithrombotique chez un groupe d'oligosaccharides inférieurs (ce terme "inférieur" étant entendu par rapport aux pentasaccharides).

Le prolongement de ces recherches a alors conduit à élaborer un groupe spécifique d'oligosaccharides qui s'est révélé doté d'une manière avantageuse d'un large spectre thérapeutique.

L'invention a donc pour but de fournir un nouveau groupe d'oligosaccharides à chaînes courtes dont la structure correspond à celle de fragments de chaînes de mucopolysaccharides acides, ou comprend de tels fragments.

Elle a également pour but les applications biologiques de ces oligosaccharides comme réactifs de laboratoire et comme principe actif de médicaments.

Les oligosaccharides de l'invention sont caractérises en ce qu'ils comprennent de 4 à 12 motifs choisis parmi les motifs sucre aminé et acide uronique, ou l'inverse, et qu'ils contiennent un enchaînement tétrasaccharidique de structure DEFG répondant à la formule II :

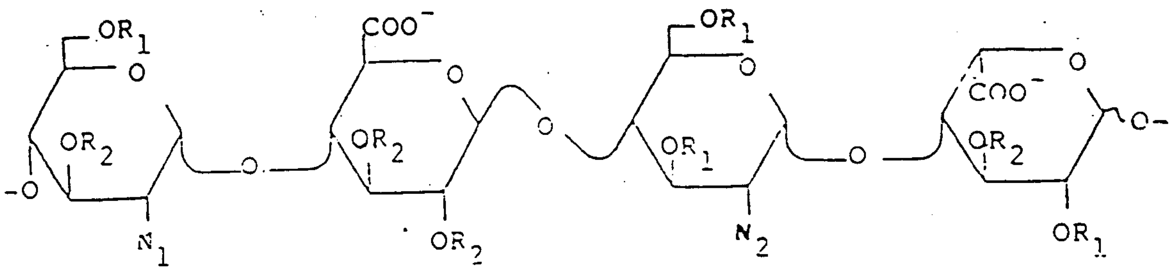

dans laquelle :

- les radicaux $R_1$, identiques ou différents les uns des autres, représentent un anion minéral estérifiant le groupe OH en position 6, en particulier un groupe sulfate ou un groupe phosphate,
- $R_2$ présente l'une des significations données pour $R_1$ ou représente un atome d'hydrogène,
- $N_1$ et $N_2$, identiques ou différents l'un de l'autre, représentent un groupe fonctionnel amine éventuellement substitué par un groupe acyle $-COR_3$ où $R_3$ représente un radical alcoyle, ou un groupe sulfoamino ou phosphoamino.

EP-A-27089 décrit des fractions oligosaccharidiques obtenues par un procédé de dépolymérisation. Des compositions renfermant la structure DEFG sont bien prévues dans ce document. Mais ces compositions ne peuvent être qu'hétérogènes étant donné les moyens d'obtention décrits.

Les travaux effectués par les inventeurs dans ce domaine ont montré l'importance de la séquence DEFG qui correspond au bloc dit irrégulier présent dans la molécule naturelle d'héparine. Cette séquence confère aux oligosaccharides et d'une manière surprenante et avantageuse aux tétrasaccharides DEFG *per se*, des propriétés pharmacologiques exploitables dans un large domaine thérapeutique.

Selon un mode de réalisation de l'invention, les oligosaccharides définis ci-dessus sont du type a - b, a-c, ou l'inverse (étant entendu qu'ils comportent la séquence DEFG).

Dans un groupe d'oligosaccharides, la chaîne glycosidique est constituée en dehors de la séquence DEFG par un seul type de ces enchaînements binaires.

Dans un autre groupe, plusieurs de ces types d'enchaînements binaires sont présents.

Selon une variante, ces oligosaccharides renferment un ou plusieurs motifs consécutifs a ou encore b ou c.

Selon une autre variante, ces oligosaccharides renferment un ou plusieurs motifs de sucres neutres et ou plusieurs désoxy-sucres dans leur structure.

Les motifs constitutifs ci-dessus sont reliés entre eux par des liaisons de type 1-2, 1-3, 1-4 ou 1-6, et, dans le cas d'oligosaccharides possédant la structure de fragments d'héparine ou d'héparane-sulfate, comportent des liaisons c 1 $\xrightarrow{\alpha}$ 4a, a 1 $\xrightarrow{\alpha}$ 4b, a 1 $\xrightarrow{\alpha}$ 4c et b 1 $\xrightarrow{\beta}$ 4a.

D'une manière inattendue, les tétrasaccharides de structure DEFG *per se* se sont révélés dotés de propriétés pharmacologiques suffisamment importantes pour permettre une utilisation de ces produits en tant que principe actifs de médicaments.

L'invention vise donc, selon un mode de réalisation préférée, les tétrasaccharides de structure DEFG, de formule II ci-dessus, en tant que tels.

On mesurera l'intérêt économique de ces produits par rapport aux oligosaccharides supérieurs dans la

mesure où ils n'impliquent pour leur synthèse que la mise en oeuvre de quatre motifs.

Une famille x de tétrasaccharides préférés en raison de leur activité anti-Xa élevée et de leur forte affinité pour l'ATIII répondent à la formule II ci-dessus dans laquelle $R_2$ représente un anion minéral.

Des tétrasaccharides de cette famille dans lesquels $R_2$ représente un anion sulfate sont plus spécialement préférés étant donné leur analogie avec les produits naturels.

A cet égard, des tétrasaccharides particulièrement intéressants renferment également des groupes sulfate pour au moins certaines de leurs autres substitutions ou plus spécialement pour touter les substitutions $R_1$ et/ou $N_1$ et $N_2$.

Un tétrasaccharide préféré de ce type correspond au produit 12 dans l'exemple 3, de formule III :

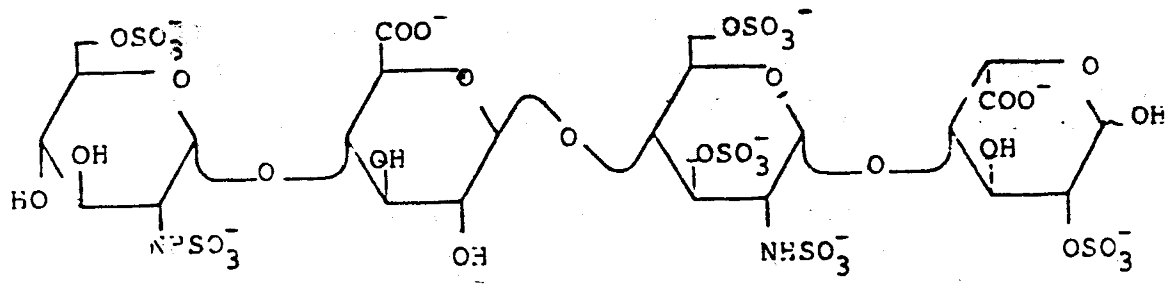

A la place des groupes sulfate, d'autres anions minéraux peuvent être présent tels que le groupe phosphate.

Une autre famille y de tétrasaccharides préféré, en particulier en raison de son activité fibrinolytique comprend un substituant $R_2$ représentant un groupe -OH.

Des tétrasaccharides de ce type comportent avantageusement des groupes sulfate pour au moins certaines de leurs autres substitutions $R_1$ et/ou $N_1$ et $N_2$. Un tétrasaccharide de ce groupe répond à la formule IV :

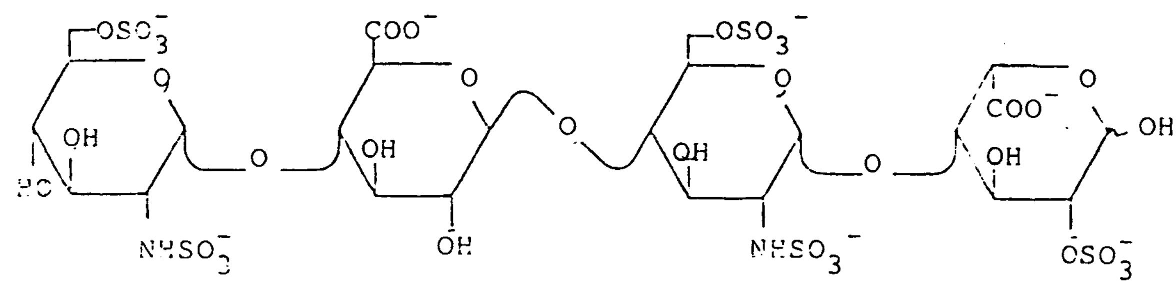

Comme dans la famille x, des produits avantageux renferment à la place d'un ou de plusieurs groupes sulfate, ou encore de la totalité de ces groupes, d'autres anions minéraux, tels que des anions phosphate.

Dans les différents types de tétrasaccharides évoqués ci-dessus, les différents anions minéraux et les groupes carboxyle se présentent avantageusement sous forme de sels avec un cation minéral en particulier un cation métallique, notamment, un cation alcalin tel que le sodium, le magnésium ou le calcium, ou encore un cation dérivé d'une base organique azotée, tel que le cation triéthylammonium.

Les dispositions qui précèdent concernant l'enchaînement DEFG s'appliquent également dans le cas où la séquence est impliquée dans une chaîne plus longue d'oligosaccharides pouvant comporter jusqu'à 12 motifs, comme défini ci-dessus.

Dans ces oligosaccharides, la séquence DEFG peut se trouver en début, incorporée, ou en fin de chaîne.

Ainsi, la séquence DEFG se trouve dans certains de ces oligosaccharides en début de chaîne, étant entendu que le pentasaccharide DEFGH No 50 dont question ci-dessus, qui est expressément décrit dans la demande de brevet principal, est exclu de la portée de la présente invention.

Les motifs qui s'enchaînent à la suite de la séquence DEFG sont tels que définis ci-dessus.

Parmi les oligosaccharides de ce type préférés en raison de l'intérêt de leur activité fibrinolytique, on citera les tétrasaccharides de la famille y (dans laquelle le motif F comporte un groupe -OH en position 3) dans laquelle la séquence DEFG est suivie d'un motif D-glucosamine.

Des pentasaccharides préférés de ce groupe comprennent ceux de structure DEFGH répondant à la formule V :

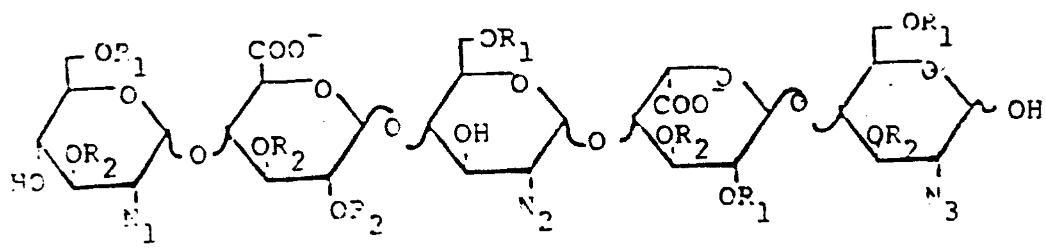

dans laquelle les substituants présentent les significations données ci-dessus et $N_3$ présente les significations données pour $N_1$ et $N_2$.

Un pentasaccharide préféré qui correspond au produit 26 de l'exemple 4 répond à la formule VI suivante :

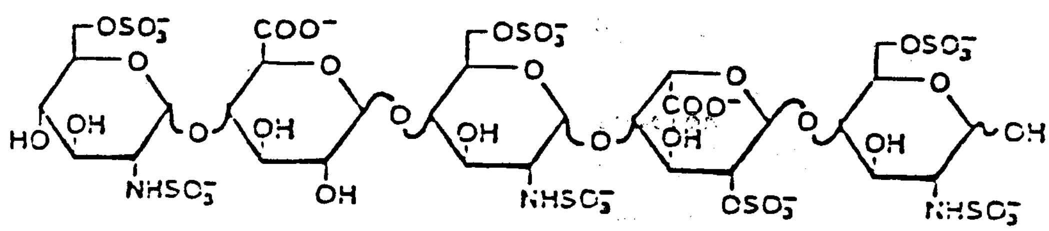

Dans d'autres oligosaccharides, l'enchaînement tétrasaccharidique DEFG se trouve incorporé dans la chaîne oligosaccharidique.

Pour d'autres oligosaccharides, cette séquence se trouve, au contraire, en bout de chaîne, les dispositions qui précèdent s'appliquent à ces deux autres groupes.

Les oligosaccharides de l'invention sont avantageusement préparés selon le procédé de synthèse décrit ci-après :

Selon la définition la plus générale de ce procédé, on fait réagir deux composés constitués ou terminés respectivement par des motifs de structure glucosamine, en particulier D-glucosamine, et des motifs de structure acide glucuronique, en particulier D-glucuronique, ou acide iduronique, en particulier L-iduronique.

L'un des motifs sucre aminé ou acide uronique est constitué par un alcool dans lequel le groupe -OH de la fonction alcool occupe l'une quelconque des positions 3,4 ou 6 dans le cas d'un motif sucre aminé et 2,3 ou 4 dans le cas d'un motif acide uronique. L'autre motif possède alors un carbone anomère activé, c'est-à-dire comportant un groupement réactif capable d'établir avec le groupe -OH de l'alcool la liaison de glycosylation -O- recherchée, selon la stéréochimie souhaitée, pour former une séquence sucre aminé-acide uronique ou l'inverse.

Les groupements présents sur les motifs mis en oeuvre pour constituer la chaîne oligosaccharidique doivent répondre notamment aux exigences suivantes :

- le groupement réactif du motif sucre aminé ou acide uronique doit être compatible avec les groupements protecteurs et/ou fonctionnels présents sur les motifs ;
- les groupements protecteurs des fonctions -OH et éventuellement des fonctions amino ou carboxyle, doivent être compatibles entre eux et avec les groupes précurseurs des fonctions amino ou carboxyle lorsqu'ils sont présents ;
- les groupes protecteurs et précurseurs doivent être inertes vis-à-vis de la réaction de glycosylation et avec les groupes réactifs, autorisant la mise en place, au cours d'opérations ultérieures, de substituants donnés aux diverses positions et ce, le cas échéant, de manière séquentielle.

La réaction de glycosylation est réalisée de manière à ne pas altérer la structure des motifs de ces produits et la nature des divers substituants présents.

L'étape de glycosylation ci-dessus est répétée de manière à obtenir la longueur de chaîne désirée.

Pour pouvoir effectuer cet allongement du squelette glucidique, on met alors en oeuvre des motifs sucre aminé ou acide uronique comportant des groupements protecteurs temporaires, c'est-à-dire des groupements capables de bloquer sélectivement une position du motif sucre aminé ou acide uronique

destinée à intervenir dans une nouvelle réaction de glycosylation. Il s'agit de groupements éliminables en présence des autres groupements présents sur les motifs en recréant un alcool.

Après élaboration du squelette glucidique recherché, on soumet la chaîne formée à une ou plusieurs réactions chimiques afin d'introduire un type de groupements fonctionnels donnés ou successivement, plusieurs types de groupements puis de former, si on le désire, des dérivés de ces groupements fonctionnels.

Pour introduire des substitutions spécifiques, c'est-à-dire des substitutions déterminées en des positions données, on utilise avec avantage des produits de départ renfermant plusieurs types de groupements protecteurs, à savoir (1) un ou plusieurs groupements semi-permanents et (2) un ou plusieurs groupements permanents.

Les groupements semi-permanents sont éliminables en premier lieu et permettent d'introduire les groupements fonctionnels souhaités aux positions qu'ils occupent. Les groupements permanents sont au contraire capables de maintenir la protection des radicaux -OH durant l'introduction des groupements fonctionnels à la place des groupes semi-permanents.

Les produits de départ du type sucre aminé renferment en outre en position 2, un groupe azoté autorisant le maintien de la présence d'une fonction azotée durant les opérations mises en oeuvre dans le procédé. Ce groupe azoté est avantageusement constitué par des groupes tels que $-N_3$ ou $-NHCOO-CH_2-C_6H_5$, ou tout autre groupe constituant un précurseur de fonction amine ou d'un dérivé d'amine, en particulier de $-NHSO_3^-$ ou de -NH-acyle, plus spécialement de $-NH-COCH_3$.

Quant aux fonctions carboxyle des motifs acide uronique, elles sont bloquées par des groupes inertes vis-à-vis des réactions mises en jeu pour le remplacement des groupes protecteurs et éliminables en fin de synthèse pour libérer les groupes carboxyle, éventuellement aux fins de salification. Ces groupes protecteurs de fonction carboxyle sont choisis avantageusement parmi les radicaux alcoyle, ou les radicaux aryle.

Ces dispositions sont mises en oeuvre pour préparer les oligosaccharides de l'invention.

En ce qui concerne plus particulièrement l'obtention de la séquence DEFG, on fait réagir avantageusement un halogénure, plus spécialement le bromure d'un disaccharide de structure EF dont la synthèse est décrite dans la demande de brevet principal avec un alcool d'un motif de structure G.

Le disaccharide EF comporte un groupe temporaire en position 4 du motif E. Ce groupe est avantageusement choisi parmi les groupes acyle, en particulier, acétyle ou chloroacétyle.

La réaction de condensation est réalisée en milieu solvant, plus spécialement dans un solvant organique, notamment du type dichlorométhane ou dichloroéthane.

Avantageusement, on utilise un catalyseur, en général un sel d'argent ou de mercure, par exemple, le trifluorométhane sulfonate d'argent, communément appelé triflate d'argent, le carbonate d'argent, l'oxyde d'argent, le bromure mercurique ou le cyanure mercurique. On utilise également un accepteur de protons tel que la sym-collidine de même qu'un capteur pour l'eau éventuellement présente et/ou pour l'acide halogénohydrique formé, par exemple des tamis moléculaires 4 Å.

L'étude des conditions de réaction montre qu'il est approprié d'opérer à température ambiante ou encore à une température inférieure pouvant atteindre 0 ° C ou moins, sous atmosphère d'un gaz inerte tel que l'azote ou l'argon.

Après formation de la chaîne trisaccharidique de structure EFG, le groupe temporaire du motif E est éliminé selon les techniques classiques pour recréer le groupe -OH. Ce dernier est alors engagé dans une réaction de glycosylation avec l'halogénure, plus spécialement le bromure du motif de structure D.

En ce qui concerne l'élaboration de la structure DEFGH de formule V, on a avantageusement recours, selon une variante de réalisation, à une réaction de condensation d'un halogènure d'un motif E, comportant un groupe temporaire en position 4, tel que le groupe lévucinoyle ou analogue, et d'un alcool d'un motif de structure F. Les positions 1 et 6 de cet alcool sont avantageusement bloquées par un pont 1,6-anhydro, qui sera ouvert après la réaction de glycosylation.

La séquence disaccharidique de structure ET formée, après traitement afin d'activer le carbone anomère du motif F, en introduisant, par exemple, un halogénure, notamment un bromure est mise en jeu dans une réaction de condensation avec un alcool de structure GH, le tétrasaccharide formé est alors traité de manière à recréer un groupe alcool en position 4 du motif E, puis condensé avec un dérivé réactif d'un motif D, notamment un halogénure, en particulier, un bromure.

Pour préparer plus spécialement, les tétrasaccharides de formule II et les pentasaccharides de formule V dans lesquelles $R_1$ représente un groupe sulfate, $R_2$ un groupe hydroxyle et $N_1$ et $N_2$ sont identiques et représentent des groupes sulfate, on met en oeuvre des produits de départ comportant les groupements suivants.

Les groupements protecteurs des radicaux -OH de ces différents motifs destinés à être sulfatés sont protégés par des groupes acyle, en particulier, acétyle, tandis que les radicaux -OH destinés à être libérés

en fin de synthèse sont protégés par un groupe permanent tel que le groupe benzyle.

Les positions 2 des motifs sucre aminé sont substituées par des groupes tels que $N_3$ ou NH-COO-$CH_2$-$C_6H_5$ et les positions 6 des motifs acide uronique sont occupées par des groupes carboxyle protégés par un radical alcoyle, en particulier méthyle.

L'étape de fonctionnalisation de la chaîne tétrasaccharidique formée,c'est-à-dire d'introduction séquentiellement de substitutions spécifiques, est alors réalisée selon les dispositions données dans la demande de brevet principal.

Ce jeu de conditions permet de réaliser l'étape de fonctionnalisation, par exemple comme suit :

On introduit tout d'abord sélectivement les groupes sulfate après avoir éliminé les groupes -O-acétyle de blocage. Cette réaction est réalisée de manière à ne pas affecter les groupes benzyle et les groupes azotés et carboxyle présents.

A cet égard, on effectue avantageusement une réaction de saponification à l'aide d'une base forte telle que la soude.

Cette réaction est réalisée de préférence à une température inférieure à l'ambiante et plus spécialement voisine de 0 ° C.

On soumet le produit résultant de l'hydrolyse à l'action d'un agent d'alcoylation afin d'introduire, sur le groupe carboxyle, les groupes alcoyle protecteurs qui se sont trouvés éliminés lors de l'hydrolyse.

Par réaction avec un agent de sulfatation, on obtient alors l'introduction de groupes sulfate aux positions libérées par l'hydrolyse et laissées libres après l'action de l'agent d'alcoylation.

Des conditions de réaction satisfaisantes pour la conduite de la sulfatation comprennent la mise en oeuvre d'un agent de sulfatation, tel qu'un complexe triméthylamine/$SO_3^-$. Cette réaction est avantageusement réalisée en milieu solvant, plus spécialement dans un solvant tel que le diméthylformamide. De préférence, on opère à une température superieure à l'ambiante, généralement voisine de 50 ° C, ce qui correspond à une durée de réaction d'environ 12 heures.

Après l'introduction des groupes sulfate sur les fonctions alcool, o n procède à la libération des groupes -OH bloqués par les radicaux benzyle.

L'élimination de groupes benzyle est avantageusement réalisée par hydrogénation catalytique dans des conditions compatibles avec le maintien des groupes sulfate et la transformation des groupes azotés en groupes fonctionnels amine.

On opère de préférence sous pression d'hydrogène en présence d'un catalyseur du type Pd/C.

Cette réaction est avantageusement réalisée en milieu solvant organique, en particulier alcoolique, additionné d'eau.

Pour obtenir l'hydrogénation des groupes azotés précurseurs et l'élimination des radicaux protecteurs des groupes -OH, la réaction est avantageusement réalisée sur une durée d'environ 3 à 4 jours.

Comme déjà indiqué, les groupes fonctionnels amine se présentent sous forme de dérivés de type N-acétyle ou N-sulfate dans les molécules biologiquement actives considérées.

Pour former des groupes N-acétyle, on soumet le produit résultant de la réaction d'hydrogénation à l'action d'un agent d'acétylation. A cet égard, l'anhydride acétique constitue un agent particulièrement approprié.

Pour réaliser cette réaction d'acétylation sélective sans affecter les autres substituants présents sur les motifs, il convient, notamment, d'opérer à pH basique, en particulier voisin de 8 en milieu aqueux.

On peut également souhaiter former des groupes N-sulfate, ce qui peut être réalisé à l'aide d'un agent de sulfatation du type indiqué ci-dessus. Des pH supérieurs à 9, avantageusement de l'ordre de 9-10, sont utilisés pour la sulfatation.

Après la réaction de sulfatation, l'addition d'une base forte permet de libérer les groupes carboxyle.

Les produits formés peuvent être aisément salifiés à l'aide de résines échangeuses d'un cation approprié. Dans les produits naturels, le cation en particulier est constitué par du sodium. On utilise donc avantageusement des résines échangeuses de cations sodium.

On peut également former des sels de potassium, lithium, magnésium, calcium. On utilise alors une résine échangeuse de protons, puis on neutralise l'acide formé avec la base du cation.

L'invention vise également les oligosaccharides constituant des intermédiaires dans les différentes étapes du procédé de synthèse défini ci-dessus.

L'étude des actions pharmacologiques des oligosaccharides de formule I renfermant la séquence DEFG ou constitués par cette séquence a permis de mettre en évidence leur intérêt en thérapeutique.

Ils exercent, en particulier, une activité sur la fibrinolyse en augmentant le taux d'activateur de plasminogène circulant et en sensibilisant le caillot à la lyse.

Les études réalisées ont été effectuées sur différents modèles expérimentaux selon la technique décrite par Vairel et al dans Ann. Pharmaceutiques françaises, 1983, 41,No 4, p.339-353.

Ainsi, 15 mn. après l'injection par voie intraveineuse chez le lapin (anesthésie 20 mn avant l'administration) de 0,25 mg d'oligosaccharides par kg, on observe une augmentation du taux d'activateur de plasminogène dans le sang circulant.

Avec, par exemple, le tétrasaccharide DEFG de formule III, on observe une augmentation moyenne des aires de lyse de 17,80 alors qu'avec du sérum physiologique utilisé à titre de témoin, on note une diminution de 0,5. Une augmentation analogue à celle du tétrasaccharide est obtenue avec le pentasaccharide de structure VI.

Les travaux effectués sur les oligosaccharides de l'invention dans lesquels le motif de structure F comporte un groupe $-SO_3$ en position 3 ont révélé une actitivé anti-Xa nettement supérieure à celle de l'héparine et une forte affinité pour l'AT-III. Dans le cas,par exemple, du tétrasaccharide de formule III-(produit N° 11)l'activité anti-Xa mesurée avec un substrat chromogène est de 600 unités anti-Xa/mg (méthode de Teien A.M. et Lie modifiée, Thrombosis Research No 10,1937,388-410).

L'efficacité thérapeutique de ces produits a été étudiée avec des modèles animaux bien définis afin de déterminer leur pouvoir antithrombotique dans des conditions pathologiques connues.

Avec le modèle de stase modifiée du lapin (voir Thromb. and Hemost. 46, (1) 117,1981 par Andersen et al), les résultats suivants ont été obtenus.

On a administré aux lapins par voie intraveineuse 50 ug/kg de produit dissous dans une solution saline (à raison de 100 ug/kg). 5 minutes avant l'addition d'un agent thrombogène.

L'agent thrombogène est constitué soit par du sérum de lapin, soit par l'agent PCC/RVV (concentré d'un complexe pro thrombine et venin de vipère Russel). Les stases des veines jugulaires droite et gauche ont été graduées sur une échelle de 0 à 10.

On observe une protection complète (cotation O) contre les effets thrombogènes induits par le sérum de lapin et une protection partielle vis-à-vis de l'agent thrombogène PCC/RVV (cotation 5), le contrôle ayant une cotation de 10.

Les oligosaccharides de l'invention dans lesquels le motif de structure F comporte, en position 3, un groupe -OH n'apparaissent pas dotés d'activité vis-à-vis du facteur Xa et présentent donc une plus grande spécificité que le groupe d'oligosaccharides avec un groupe $-SO_3^-$ en position 3 du motif de structure F vis-à-vis de l'activité fibrinolytique.

Les études toxicologiques des produits de l'invention ont montré leur innocuité ce qui les rend précieux pour l'élaboration de médicaments.

L'invention est donc également relative à des préparations pharmaceutiques qui renferment lesdits oligosaccharides.

Elle concerne plus particulièrement des préparations pharmaceutiques dépourvues de substances pyrogènes, contenant une quantité efficace de principes actifs en association avec des excipients pharmaceutiques.

Elle vise notamment les compositions dans lesquelles le véhicule pharmaceutique est approprié pour l'administration par voie orale. Des formes d'administration de l'invention appropriées pour l'administration par voie orale peuvent être avantageusement des gélules gastrorésistantes, des comprimés ou tablettes, des pilules, ou encore présentées sous forme de liposomes.

D'autres compositions pharmaceutiques comprennent ces oligosaccharides en association avec les excipients appropriés pour l'administration par voie rectale. Des formes d'administration correspondantes sont constituées par des suppositoires.

D'autres formes d'administration de l'invention sont constituées par des aérosols ou des pommades.

L'invention concerne également des compositions pharmaceutiques injectables, stériles ou stérilisables pour l'administration tant par voie intraveineuse qu'intramusculaire ou sous-cutanée. Ces solutions renferment avantageusement les produits de la famille x à raison de 1000 à 1OO OOO u (Yin-Wessler)/ml d'oligosaccharides, de préférence de 5OOO à 5O OOO, par exemple de 25 OOO u/ml, lorsque ces solutions sont destinées à l'injection par voie sous-cutanée. Elles peuvent contenir, par exemple, de 5OO à 1O OOO, notamment 5OOO u/ml d'oligosaccharides lorsqu'elles sont destinées à l'injection par voie intraveineuse ou par perfusion.

Avantageusement, de telles préparations pharmaceutiques sont présentées sous la forme de seringues non récupérables, prêtes à l'emploi.

L'invention concerne également les compositions pharmaceutiques contenant lesdits oligosaccharides en association avec un autre principe actif.

Les compositions pharmaceutiques de l'invention sont particulièrement adaptées pour le contrôle (préventif ou curatif) de certaines étapes de la coagulation du sang chez l'homme ou l'animal, notamment dans le cas où le patient est soumis à des risques d'hypercoagulabilité résultant notamment d'opérations chirurgicales, de processus athéromateux, de développement de tumeurs et de troubles de la coagulation

par des activateurs bactériens ou enzymatiques, etc.

Afin d'illustrer l'invention, on indique, ci-après un exemple de posologie utilisable chez l'homme avec les produits de la famille x : cette posologie comprend, par exemple, l'administration au patient de 1000 à 25 000 u (Yin et Wessler) par voie sous-cutanée, une à trois fois par jour, selon le niveau des risques d'hypercoagulabilité ou la condition thrombotique du patient, ou de 1000 à 25OOO u/24 heures, par voie intraveineuse, en administrations discontinues à intervalles réguliers, ou continues par perfusion, ou encore de 1000 à 25000 u (trois fois par semaine) par voie intramusculaire ou sous-cutanée (ces titres sont exprimés en unités Yin-Wessler). Ces doses peuvent être naturellement ajustées pour chaque patient en fonction des résultats et des analyses de sang effectuées auparavant, la nature des affections dont il souffre et, d'une manière générale, son état de santé.

Dans le cas des produits de la famille y, notamment des pentasaccharides de formule VI, on administre 1 à 100 mg/jour selon l'état du patient et la forme pharmaceutique utilisée.

Outre les compositions pharmaceutiques renfermant les oligosaccharides tels quels, l'invention vise également les compositions pharmaceutiques renfermant au moins un oligosaccharide tel que défini ci-dessus, conjugué, par liaison covalente, à un support soluble ou à un support insoluble avantageusement au moyen du sucre terminal réducteur.

Des conjugués fixés à des supports solubles préférés sont constitués plus spécialement d'oligosaccharides AT-III conjugués comportant une séquence DEFG de formule II, plus spécialement un groupe sulfate. De tels produits constituent des médicaments particulièrement intéressants dans la prévention de thromboses, en cas de déficits en AT-III.

D'autres conjugués préférés avec supports solubles sont formés d'un oligosaccharide de formule générale II, fixé à un véhicule tel qu'une protéine, notamment, la polylysine,ou le sérum albumine bovine.

Ces produits sont utilisables comme immunogènes eux-mêmes sources d'anticorps circulants produits in vivo ou d'anticorps monoclonaux, clonés in vitro selon les techniques appropriées.

Dans d'autres conjugués préférés, les oligosaccharides de l'invention sont conjugués à des supports insolubles. On utilisera avantageusement les supports classiques. Ces conjugués, lorsqu'ils renferment une séquence DEFG selon la famille x ci-dessus sont utilisables comme immunoabsorbants, par exemple pour une purification de haute spécificité de l'AT III et pour son dosage ou pour l'élaboration, par fixation sur des polymères biocompatibles, de nouveaux polymères hémocompatibles athrombotiques.

En outre, les complexes résultant de l'affinité de l'AT III pour les oligosaccharides de l'invention renfermant la séquence DEFG de la famille x entrent également dans le cadre de l'invention.

L'invention vise également l'application des oligosaccharides considérés en médecine nucléaire, en tant que produits radiopharmaceutiques. Ces produits sont alors marqués par des traceurs choisis parmi ceux couramment utilisés dans ce domaine, et notamment à l'aide de technétium 99 m.

A cet effet, on transforme le technétium 99 m obtenu à partir de générateurs du commerce, sous forme de pertechnétate de sodium de valence 7 non réactif, en technétium réduit de valence 4 qui serait la forme la plus réactive du technétium. Cette transformation est effectuée grâce à un système réducteur réalisé à partir de sels d'étain (chlorure stanneux), de sels de fer (sulfate ferreux), de sels de titane (trichlorure de titane) ou autres sels.

La plupart du temps, cette simple réduction du technétium suffit, dans des conditions de pH données, à réaliser la fixation du technétium sur la molécule considérée.

On peut utiliser les produits de l'invention, qui constituent en quelque sorte un support, à des doses de l'ordre de 1OO à 2OO u Yin-Wessler.

Pour l'élaboration de ces réactifs radiopharmaceutiques, on peut opérer conformément à la méthode de P.V. KULKARNI et al. dans The Journal of Nuclear Medecine 21, N° 2, p. 117-121.

Les produits ainsi marqués sont avantageusement utilisés dans des tests in vivo pour la détection et le diagnostic d'extension des thromboses et des états thrombotiques.

Les oligosaccharides de l'invention peuvent être également utilisés pour la détermination de la spécificité des nombreuses enzymes impliquées dans le métabolisme des glycosaminoglucuronoglycanes.

D'autres caractéristiques avantageuses de l'invention apparaîtront dans les exemples qui suivent et en se reportant aux figures 1 à 5 illustrant les produits mis en oeuvre dans les synthèses décrites.

Dans ces figures, les références numériques des formules sont utilisées également dans les exemples pour désigner les mêmes produits.

Les abréviations utilisées dans ces formules présentent les significations suivantes :

Ac : un groupe acétyle ; Me : méthyle ; Bn : benzyle ;
Lev : levucinoyle ; MCA : monochloroacétyle et Z un groupe benzyloxycarbonyle.

EXEMPLE 1 - Préparation du trisaccharide 3 de structure EFG, à savoir du benzyl-O-[méthyl-2,3-di-O-benzyl-4-O-chloroacétyl-β-D-glucopyranosyluronate-(1→4)-O-(3,6-di-O-acétyl-2-azido-2-désoxy-α-D-glucopyranosyl)-(1→4)-O-[méthyl - 2-O-acétyl-3-O-benzyl-α-L-idopyranosiduronate].

On effectue la synthèse du trisaccharide 3 par condensation d'un halogénure de structure EF et d'un alcool de structure G. Ces réactifs sont numérotés respectivement 1 et 2 dans la description.

Cette étape de condensation est réalisée comme suit :

Une solution de l'halogénure 1 (738 mg ; 0, 92 mmole) et de l'alcool 2 (428 mg ; 1 mmole) dans le dichloroéthane anhydre (15 ml) est agitée à -20°C en présence de tamis moléculaires 4 Å en poudre. On ajoute ensuite de la collidine (150 µl) puis du triflate d'argent (262 mg ; 10 mmole). Après une heure à -20°C le mélange réactionnel est dilué avec du dichlorométhane puis filtré. La phase organique est lavée ($KHSO_4$) 10% ; eau), séchée ($Na_2SO_4$) puis concentrée à sec. La mousse obtenue (1,07 g) est chromatographiée sur gel de silice (toluène/acétate d'éthyle ; 4/1 ; v/v) livrant ainsi le trisaccharide 3 pur (699 mg ; 63%) sous forme de mousse blanche.

$[\alpha]_D$ : + 25° (c 1,4 ; chloroforme).

EXEMPLE 2 - Préparation d'un tétrasaccharide 6 de structure DEFG, à savoir le benzyl O-(6-O-acétyl-2-azido-3,4-di-O-benzyl-2-désoxy-α-D-glucopyranosyl)-(1→4)-O-[méthyl 2,3-di-O-benzyl-β-D-glucopyranosyluronate]-(1→4)-O-(3,6-di-O-acétyl-2-azido-2-désoxy-α-D-glucopyranosyl)-(1→4)-O-(méthyl 2-O-acétyl-3-O-benzyl-α-L-idopyranosiduronate).

Cette synthèse est réalisée par condensation d'un halogénure 5 de structure D avec un alcool 4 correspondant au trisaccharide 3 dont le groupe -OH en position 4 du motif E a été débloqué.

On décrit donc successivement :

a) l'obtention du trisaccharide 4 à partir du trisaccharide 3,

b) la condensation de 4 avec l'halogénure 5.

Etape a :

Le trisaccharide 3 (669 mg : 0,55 mmole) est dissous dans un mélange de lutidine (3,8 ml) et d'acide acétique (1,25 ml). Du méthanol est ensuite ajouté suivi d'une solution d'hydrazine dithiocarbonate obtenue selon la méthode décrite par van Boeckel et Beetz dans Tetrahedron letters 24 (1983) 3775-3778. Après deux heures à température ambiante la solution est diluée par addition de dichlorométhane (200 ml) puis lavée ($NaHCO_3$ saturé, eau; $KHSO_4$ 10%, eau), séchée ($Na_2SO_4$) et concentrée à sec. Le produit pur (530 mg ; 83%) est obtenu après chromatographie sur gel de silice (toluène/acétate d'éthyle ; 2/1 ; v/v).

$[\alpha]_D$ : + 29° (c 1,29 ; chloroforme).

| Analyse : | |
|---|---|
| Calculé pour $C_{54}H_{11}O_{20}N_3$ | Trouvé |
| C 60,50 | 60,46 |
| H 5,74 | 5,74 |
| N 3,92 | 4,11 |

Etape b :

Une solution d'alcool 4 (494 mg ; 0,455 mmole) dans le dichlorométhane et de l'halogénure 5 (1,096 g ; 2,2 mmoles) est traitée par le triflate d'argent (632 mg) en présence de sym collidine (360 µl) comme il est décrit pour la synthèse du composé 3. Après purification sur colonne de gel de silice (gradient chloroforme → chloroforme acétate d'éthyle : 20/1 : v/v) on obtient le dérivé 6 pur (595 mg: 89%).

$[\alpha]_D$ : + 43° (1,27 ; chloroforme).

| Analyse : | |
|---|---|
| Calculé pour $C_{76}H_{84}O_{24}N_6$ | Trouvé |
| C 61,61<br>H 5,71<br>N 5,67 | 61,57<br>5,76<br>5,61 |

EXEMPLE 3 - Préparation du tétrasaccharide 11 de structure DEFG, à savoir le O-(2-désoxy-6-O-sulfo-2-sulfamido-α-D-glucopyranosyl)-(1→4)-O-(β-D-glucopyranosyluronate)-(1→4)-0-(2-désoxy-3,6-di-O-sulfo-2-sulfamido-α-D-glucopyranosyl)-(1→4)-O-2-O-sulfo-α-L-idopyranuronate, octa sel de sodium.

On obtient le tétrasaccharide par déblocages séquentiels des groupes -OH et introduction des groupes désirés sur le tétrasaccharide 6.

Les étapes successives mises en oeuvre sont les suivantes :

1 - libération des groupes -OH bloqués par des groupes acétyle ;

2 - formation des sels de sodium des groupes sulfates ;

3 - formation des sels de sodium des groupes carboxyle ;

4 - libération des groupes -OH bloqués par des groupes benzyle et transformation des groupes azido en groupes amino,

5 - estérification des fonctions aminés .

Ces étapes sont réalisées comme suit :

1 - Passage de -OAc à -OH conduisant au tétrasaccharide de 7.

Le dérivé 6 (0,340 g) est dissous dans un mélange de méthanol (30,6 ml) de chloroforme (3,5 ml) et d'eau (4,25 ml). De la soude 5N est alors ajoutée (4,25 ml). Après 4 heures à température ambiante, on ajoute du chloroforme (90 ml) puis de l'acide chlorhydrique (8N ; 8 ml). Après décantation la phase chloroformique est séparée. La phase aqueuse est lavée par le chloroforme (3 x 10 ml). Les phases chloroformiques sont regroupées et séchées ($Na_2SO_4$).Le résidu obtenu après concentration à sec est méthylé par le diazométhane, puis il est chromatographié sur colonne de gel de silice éluée par un gradient (dichlorométhane → dichlorométhane/acétate d'éthyle ; 1/1 ; v/v). On obtient le composé 7 - (0,173 g : 57%).

$[\alpha]_D$ : 14° (c l ; chloroforme).

| Analyse : | |
|---|---|
| Calculé pour $C_{68}H_{76}O_{21}N_6$, 0,5 $H_2O$ | Trouvé |
| C 61,68<br>H 5,93<br>N 6,34 | 61,62<br>5,84<br>6,31 |

2 - Passage de -OH à $-O-SO_3Na$ conduisant au tétrasaccharide 8.

La composé 7 (117 mg ; 0,068 mmoles) est dissous dans du diméthylformamide anhydre (3 ml) puis il est sulfaté pendant une nuit à 50°C en présence de triméthylamine/$SO_3$ (123 mg). Le mélange réactionnel est alors dilué par addition d'un mélange méthanol chloroforme (1/1 ; v/v ; 3 ml) puis il est chromatographié sur une colonne de Séphadex LH 20 équilibrée dans chloroforme/méthanol (1/1 ; v/v). Le produit obtenu est utilisé directement pour la préparation de 9.

3 - Passage de COOMe à COONa, conduisant au tétrasaccharide 9.

Le composé 8 (357 mg ; 0,207 mmole) est dissous dans un mélange de méthanol (8 ml) et d'eau (2 ml), puis on ajoute de la soude (5N ; 1,2 ml). Après 3 heures de réaction le mélange réactionnel est passé au travers d'une colonne de résine Dowex 50 - $H^+$ équilibrée dans un mélange méthanol/eau (8/2 ; v/v). L'éluat est passé au travers d'une colonne Dowex 50 - $Na^+$ équilibrée dans le même solvant. Après évaporation à sec le produit 9 est obtenu pur(0,267 g) %) à la suite d'une chromatographie sur un gel de silice (30 g ; acétate d'éthyle/pyridine/acide acétique/eau ; 160/77/19/42 ; v/v/v/v), suivie d'un passage sur l'échangeur d'ions Séphadex SPC 25 (18 g) équilibrée sous forme $Na^+$ dans un mélange méthanol/eau (8/2 ; v/v).

$[\alpha]_D$ : 5,50 ( 1,025 ; méthanol).

4 - Passage de -OBn à -OH et de $-N_3$ à $-NH_2$ conduisant au tétrasaccharide 10.

Le composé 9 (256 mg ; 0,147 mmole) est dissous dans un mélange méthanol/eau (9/1 ; v/v ; 10 ml) puis hydrogéné en présence d'un catalyseur (Pd/C 10%); 130 mg) pendant 5 jours. Le catalyseur est remplacé par du catalyseur frais puis la réaction est continuée pendant 4 jours. Le spectre U.V. du produit montre alors l'absence de dérivés benzylés. Le produit obtenu (0,17 g) est envoyé directement dans la préparation de 11.

5 - Passage de $-NH_2$ à $-NHSO_3Na$ conduisant au tétrasaccharide 11.

A une solution de 10 (0,17 g ; 0,152 mmoles) dans l'eau (10 ml) on ajoute peu à peu le complexe pyridine/$SO_3$ (140 mg ; 0,75 mmole) tout en maintenant le pH à 9,5 par addition de soude 2M. Une nouvelle addition de complexe sulfatant est faite après 1 heure (70 mg) et 1 nuit (70 mg). Le mélange réactionnel est alors déposé au sommet d'une colonne de séphadex G 50 (300 x 2,5 cm) éluée par du chlorure de sodium 0,2 M. Le produit est ensuite absorbé au sommet d'une colonne de résine Biore AG 1x2 (1,6 x 10 cm) sous forme $Na^+$ éluée par un gradient de chlorure de sodium (0,5 M → 3M). Les fractions contenant le produit sont regroupées. Les sels sont éliminés par chromatographie sur séphadex G-25 (100 mg). Le produit est ensuite lyophilisé (111 mg ; 54 %).

$[\alpha]_D$ : + 46 (c 0,85 ; eau).

En mettant en oeuvre dans les étapes qui précèdent un motif G comportant un groupe $-OCH_3$ à la place du groupe -OBn sur le carbone anomère, on obtient le tétrasaccharide 12.

On indique dans le tableau ci-après les significations des substituants $A_1$ à $A_5$ de formule 5 bis donnée sur la planche I pour les composés 6 à 12.

| | $A_1$ | $A_2$ | $A^3$ | $A_4$ | $A_5$ |
|---|---|---|---|---|---|
| 6 | Ac | Bn | $N_3$ | Me | Bn |
| 7 | H | Bn | $N_3$ | Me | Bn |
| 8 | $So_3Na$ | Bn | $N_3$ | Me | Bn |
| 9 | $So_3Na$ | Bn | $N_3$ | $Na^+$ | Bn |
| 10 | $So_3Na$ | H | $NH_2$ | $Na^+$ | H |
| 11 | $So_3Na$ | H | $NHSO_3Na$ | $Na^+$ | H |
| 12 | $So_3Na$ | H | $NHSO_3Na$ | $Na^+$ | $CH_3$ |

EXEMPLE 4 : Préparation du pentasaccharide 27 de structure DEFGH, comportant un motif F avec en position$^3$ un groupe -OH, ou

O-(2-désoxy-6-O-sulfo-2-sulfo-amino-α-D-glucopryranosyl)-(1→4)-O-(β-D-glucopyranosyluronate)-(1→4)-O-(2-desoxy-6-O-sulfo-2-sulfo-amino-α-D-glucopyranosyl)-(1→4)-O-(2-O-sulfo-α-L-idopyranosyluronate)-(1→4)-2-désoxy-6-sulfo-2-sulfo-amino-D-glucopyranose-

Pour la préparation de ce pentasaccharide, on a recours aux étapes a) à j) suivantes :

a - Préparation du disaccharide 15 de structure EF, a savoir le

1,6-anhydro-2-azido-3-O-benzyl-2-désoxy-4-O-(méthyl 2,3 di-O-benzyl-4-O-lévulinyl-β-D-glucopyranosyluronate)-β-D-glucopyranose -

On soumet à agitation une solution de l'alcool 14 (4,7g ; 17mmoles) dans le dichlorométhane anhydre (4O ml), à l'abri de la lumière et de l'humidité, en présence de carbonate d'argent (4,3 g ; 15mmoles), de tamis moléculaire 4 Å en poudre et de drièrite Après 1 heure d'agitation, on ajoute goutte à goutte à O°C une solution de l'halogénure 13 (5,7 g ; 10,3 mmoles) dans le dichlorométhane (20ml). Après cinq jours de réaction, on dilue le mélange réactionnel par du dichlorométhane, on le filtre et on concentre à sec. Par chromatographie sur gel de silice, on récupère l'alcool n'ayant pas réagi (élué par un mélange hexane /acétate d'éthyle ; 2/1 ; v/v) et le disaccharide 15 (élué par un mélange hexane/acétate d'éthyle ; 1/1 ; v/v) on cristallise le disaccharide 15 à l'aide d'un mélange acétate d'éthyle/hexane (4,1 g : 53%).

PF : 93-94°C

$[\alpha]_D$ : +5° (c l ; chloroforme)

b - Ouverture du pont 1,6-anhydro du motif de structure F

On soumet à agitation un mélange du disaccharide 15 (4,5g), d'anhydride acétique (42ml) et d'acide trifluoroacétique (12ml) à température ambiante pendant 14 heures environ. Après évaporation à sec, le résidu obtenu est chromatographié sur gel de silice à l'aide d'un mélange hexane/acétate d'éthyle : 6/5 (v/v). On obtient le dérivé 17 (2,7 g ; 52%) ainsi que le dérivé 3-O-acétylé 16 (1,4 g).

$[\alpha]_D$ : + 15° (c l,l ; chloroforme)

c -

**Transformation du groupe -OAc en position 1 du motif de structure F en groupe -OH :**

On soumet à agitation un mélange du composé 17 (2,46 g ; 2,9 mmoles), d'éther (12O ml) et de benzylamine (12ml), à température ambiante, pendant 6 heures. Après dilution avec de l'éther (7OO ml), la solution obtenue est lavée avec HCl 1 M froid puis de l'eau et concentrée à sec. Le sirop obtenu est chromatographié sur gel de silice à l'aide d'un mélange chloroforme/acétate d'éthyle : 2/1 (v/v). On obtient ainsi le dérivé 18 (2,O47 g : 84,5%) sous forme de mousse blanche.

$[\alpha]_D$ : + 21,30° (c 1,6 ; chloroforme)

d - Bromuration de la position 1 du motif de structure F:

A une solution du dérivé 18 (162 mg ; 0,2 mmole) dans le dichlorométhane (5ml), on ajoute à O°C de la sym-collidine (320 µl) puis du bromure de N,N-diméthyl formiminium fraîchement préparé (réactif de Vilsmeier). Après 6 heures de réaction, on procède à une nouvelle addition de réactif de Vilsmeier, puis on laisse environ 14 heures à 4°C. Après dilution par du dichlorométhane, lavage à l'eau froide, séchage et évaporation, le résidu est chromatographié rapidement sur gel de silice à l'aide d'un mélange dichlorométhane/acétate d'éthyle : 5/1 (v/v). On obtient le composé 19 (83 mg : 4 7.5 %) qui est immédiatement engagé dans la réaction de glycosylation.

e - réaction de glycosylation conduisant à un enchaînement tétrasaccharidique:

On soumet à agitation une solution de l'halogénure 19 (360mg ; 0,414mmole) et de l'alcool 20 (710 mg ; O,82 mmole) dans le dichloroéthane (10 ml), à -20°C en présence de tamis moléculaire 4 Å en poudre. On ajoute de la sym-collidine (66µl) puis du triflate d'argent (117 mg;O,45 mmole). Les mêmes quantités de sym-collidine et de triflate d'argent sont ajoutées après 2 heures de réaction, puis on laisse le mélange revenir à O°C. Après 14 heures environ, on procède à une nouvelle addition de triflate et de collidine. La réaction est terminée au bout de 48 heures. Après dilution par le dichlorométhane, le mélange réactionnel est filtré. La solution est lavée ($KHSO_4$ 10%, eau), séchée ($Na_2SO_4$) et concentrée à sec. Le sirop obtenu (1 g) est chromatographié sur gel de silice (dichlorométhane/acétate d'éthyle • 4,5/1 ; v/v) donnant le dérivé 21 attendu (287 mg ; 42,5%) ainsi que l'alcool 20 n'ayant pas réagi (990 mg ; 55%).

$[\alpha]_D$ : +45,60° (c l,78 ;chloroforme).

f - Transformation du groupe -OLev en position 4 du motif E en groupe -OH :

A une solution du composé 21 (272 mg ; O,165mmole) dans la pyridine (0,9ml), on ajoute une solution 1M d'hydrate d'hydrazine dans un mélange pyridine/acide acétique (3/2 ; v/v). Après 5 minutes, le mélange est dilué par du dichlorométhane puis la solution est lavée ($KHSO_4$ 10%, eau, $NaHCO_3$ saturé, eau),séchée ($Na_2SO_4$) et concentrée à sec. Le résidu solide obtenu (269mg) est soumis à une chromatographie sur colonne de gel de silice. On utilise un mélange hexane/acétate d'éthyle : 1/1 (v/v). On obtient ainsi le dérivé 22 (219 mg ; 86%).

$[\alpha]_D$ : + 51,50° (l,05 ; chloroforme).

g -

**Réaction de glycosylation avec le tétrasaccharide 21 et le monosaccharide 5 de structure D.**

On soumet à agitation, à température ambiante, pendant 30 mn, en présence de tamis moléculaire 4 Å en poudre, une solution du composé 22 (208 mg ; O,134 mmole) et de l'halogénure 5 (350mg ; O,67 mmoles) dans le dichloroéthane (8ml).

Après refroidissement à -20°C, on ajoute de la sym-collidine (114 µl) puis du triflate d'argent (201 mg ; 0,74 mmoles). Après une heure le mélange réactionnel est dilué par du dichlorométhane et filtré. La solution est lavée ($KHSO_4$ 10%, eau), séchée ($Na_2SO_4$) et concentrée à sec. Le résidu obtenu (410mg) est chromatographié sur gel de silice donnant le pentasaccharide 23 (203 mg ; 77,5%) sous forme de mousse.

$[\alpha]_D$ : + 57 (c l; chloroforme).

h - Libération des groupes -OH bloqués par des groupes acétyle:

Le composé 23 (193 mg ; O,O98mmole) est dissous dans un mélange de chloroforme (5ml), de méthanol (18ml) et d'eau (2,5ml). On ajoute alors goutte à goutte une solution de soude 5N (2,5ml).

Après 7 heures de réaction, le mélange réactionnel est dilué le chloroforme (50ml) puis acidifié par addition d'acide chlorhydrique aqueux. Le produit est extrait par le chloroforme. La phase chloroformique est lavée à l'eau jusqu'à pH neutre. Le produit est alors méthylé par addition de diazométhane. Le sirop obtenu après évaporation à sec est purifié sur une colonne de gel de silice (Lobas Merck, type A) éluée par un gradient (chloroforme → méthanol/chloroforme ; 1/40 ; v/v). On obtient ainsi le composé 24 pur (96 mg ; 55%).

$[\alpha]_D$ ; + 45° (c 1 ; chloroforme).

i - Sulfatation des groupes -OH libérés:-

Le composé 24 (88 mg ; O,O49mmole), dissous dans du DMF anhydre (1,5 ml) est sulfaté pendant environ 14 heures à 50°C par le complexe triméthylamine /$SO_3$ (72mg,0,5mmole). Le mélange est alors dilué par addition de chloroforme (O,75ml) et de méthanol (0,75ml),puis chromatographié sur une colonne de gel Séphadex LH-LO éluée par un mélange chloroforme/méthanol (1/1 ; v/v). Les fractions contenant le dérivé 25 sont regroupées, concentrées et le produit est purifié sur gel de silice (acétate d'éthyle/pyridine/acide acétique eau ; 160/77/19/42 ; v/v/v/v). On obtient ainsi le dérivé 25 après passage sur échangeur d'ions Séphadex SP25 $Na^+$. Ce produit se présente sous forme d'une poudre blanche (97mg ; 89%).

$[\alpha]_D$ : 36° (c l ; méthanol).

j - Libération des groupes -OH bloqués par les groupes benzyle, transformation des groupes -$N_3$ en groupes -$NHSO_3$ et des groupes -COOMe en groupes -$COO^-$.

Le composé 25 (55 mg ; O,O25mmole) est hydrogéné dans un mélange méthanol/eau (9/1) en présence de catalyseur (Pd/C , 5% ; 50 mg). Après 8 jours l'hydrogénation est complète. Après filtration et concentration à sec, le produit obtenu est dissous dans l'eau, le pH est amené à 9,5, puis maintenu à cette valeur pendant toute la durée de la réaction. On ajoute alors du complexe pyridine/$SO_3$ aux temps 0 (54mg), 30 minutes (27mg) et 1 heure (27mg). Après une nuit, le mélange réactionnel est déssalé au moyen d'une colonne de gel de Séphadex G 50 (1,8x4Ocm) équilibrée dans l'eau. Les fractions contenant le produit 26 sont concentrées puis le résidu est déposé sur une colonne (1,25x145cm) de Séphadex G 25 éluée par du chlorurede sodium 0,2M. Les fractions contenant le pentasaccharide 26 sont passées au travers d'un échangeur d'anions (Biorex AG 1x2 $Cl^-$ ; 1,6 x 15 cm). Le dérivé 26 est ensuite élué par un gradient de chlorure de sodium (O,5 → 3M) Il est obtenu pur après dessalage (Séphadex G 25) et lyophilisation. C'est une poudre légère blanche (10mg ; 30%).

$[\alpha]$ : +40° (c l ; eau).

Les caractéristiques spectrales en RMN sont les suivantes :

$^1$H-RMN :$\delta$ dans $D_2O$ par rapport au TSP interne-

Unité D : 5,64(H-1) ; 3,30 (H-2) ; 3,63 (H-3) ; 3,57 (H-4) ; 3,90 (H-5) ; 4-4,5 (H-6,6) ;

Unité E : 4,62 (H-1) ; 3,40 (H-2) ; 3,87 (H-3) ;

Unité F : 5,45 (H-1) ; 3,25-3,30 (H-2) ;

Unité G : 5,24 (H-1) ; 4,33 (H-2) ;4,20 (H-3) ; 4,11 (H-4) ; 4,81 (H-5) ;

Unité H : 5,44 (H-1$\alpha$) ; 4,70 (H-1$\beta$) ; 3,25 (H-2$\alpha$) ; 3,O5 (H-2$\beta$) ; 3,69 (H-3) ; 3,79 (H-4).

Le spectre RMN $^1$H de ce tétrasaccharide est rapporté sur la figure 5, dans la partie inférieure. A titre de comparaison, on a indiqué dans la partie supérieure le spectre de RMN du pentasaccharide correspondant comportant un groupe -$OSO_3^-$ en position 3 du motif F. On attribue le signal à 5,51 ppm observé avec DEFGH comportant le groupe -$OSO_3^-$ sur le motif F en position 3 au proton anomère du motif glucosamine 3-O-sulfate. Ce signal est déplacé à environ 5,45 ppm pour le motif F correspond non sulfaté et se superpose au signal correspondant du motif H.

## Revendications

1. Oligosaccharides possédant la structure de fragments de chaînes de mucopolysaccharides acides ou comprenant de tels fragments, caractérisés en ce qu'ils comprennent de 4 à 12 motifs choisis parmi les motifs sucre aminé et acide uronique, ou l'inverse, et qu'ils contiennent un enchaînement tétrasaccharidique de structure DEFG répondant à la formule II :

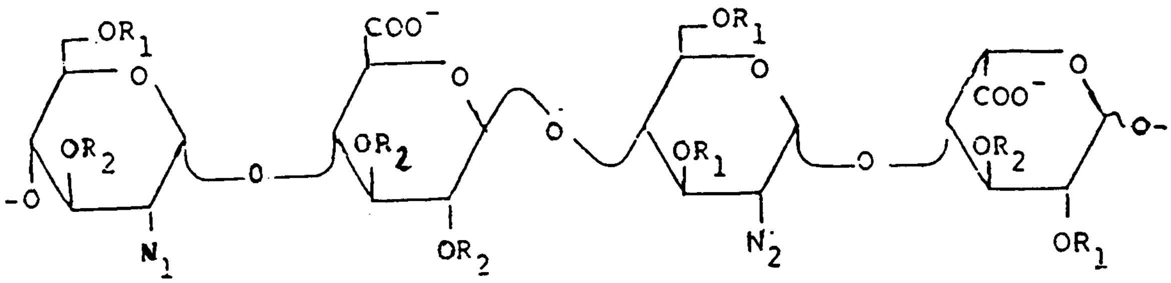

dans laquelle :

- les radicaux $R_1$, identiques ou différents les uns des autres, représentent un anion minéral estérifiant le groupe -OH en position 6, en particulier, un groupe sulfate ou un groupe phosphate,
- $R_2$ présente l'une des significations données pour $R_1$ ou représente un atome d'hydrogène,
- $N_1$ et $N_2$, identiques ou différents l'un de l'autre, représentent un groupe fonctionnel amine, éventuellement substitué par un groupe acyle $-COR_3$ où $R_3$ représente un radical alcoyle ou un groupe sulfoamino ou phosphoamino, et leurs sels,

à l'exclusion du pentasaccharide de formule :

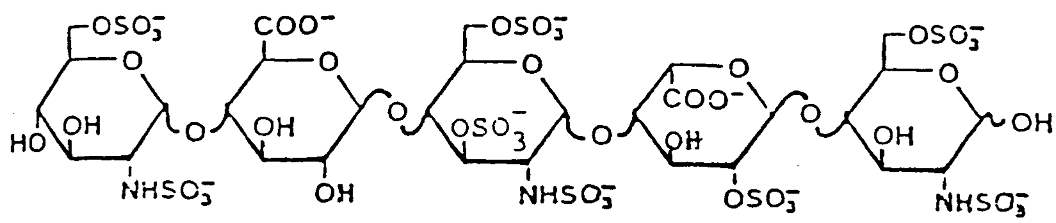

**2.** Oligosaccharides selon la revendication 1, caractérisés en ce qu'ils sont constitués, en dehors de la séquence DEFG, par des motifs binaires D-glucosamine acide D-glucuronique, a-b, D-glucosamine acide L-iduronique a-c, ou l'inverse, par un seul type de ces enchaînements binaires, par plusieurs de ces types d'enchaînements, ou qu'en variante ils renferment un ou plusieurs motifs consécutifs a, b, ou c, ou encore qu'ils renverment un ou plusieurs motifs de sucres neutres et/ou plusieurs désoxy-sucres dans leur structure, ces motifs étant reliés entre eux par des liaisons de type 1-2, 1-3, 1-4 ou 1-6 et, dans le cas d'oligosaccharides possédant la structure de fragments d'héparine ou d'héparane-sulfate, par des liaisons c 1

$\xrightarrow{\alpha}$

4a, a 1

$\xrightarrow{\alpha}$

4b, a 1

$\xrightarrow{\alpha}$

4c et b 1

$\xrightarrow{\beta}$

4a.

3. Les tétrasaccharides DEFG de formule II per se.

4. Tétrasaccharides selon la revendication 3, caractérisés en ce que le substituant $R_2$ en position 3 du motif F représente un anion minéral estérifiant le groupe -OH en position 3, plus spécialement un anion sulfate, au moins certaines des autres substitutions ou plus spécialement toutes les substitutions $R_1$ ou $N_1$ et $N_2$ comportant également avantageusement un groupe sulfate.

5. Tétrasaccharide selon la revendication 4, caractérisé en ce qu'il répond à la formule III :

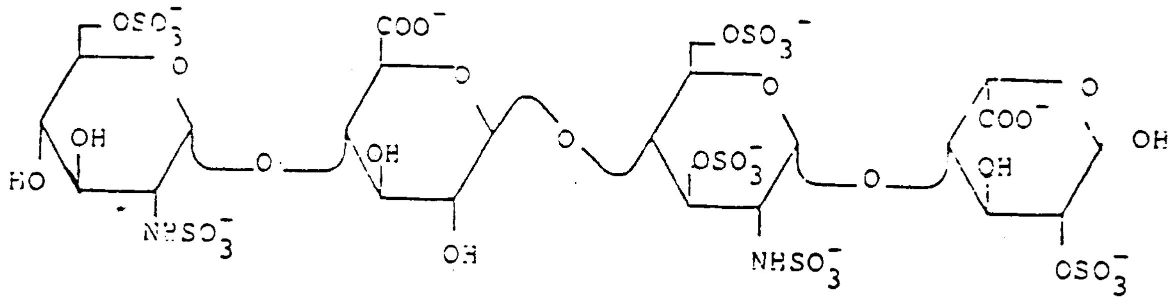

6. Tétrasaccharides selon la revendication 3, caractérisés en ce que le substituant $R_2$ en position 3 du motif F représente un groupe -OH, au moins certaines des autres substitutions $R_1$ et/ou $N_1$ et $N_2$ comportant un groupe sulfate.

7. Les oligosaccharides selon la revendication 1 renfermant un tétrasaccharide selon l'une quelconque des revendications 4 à 6.

8. Oligosaccharides selon la revendication 7, caractérisés en ce que la séquence DEFG est suivie d'un motif D-glucosamine.

9. Oligosaccharides selon la revendication 8, caractérisés en ce qu'il s'agit de pentasaccharides de structure DEFGH répondant à la formule V :

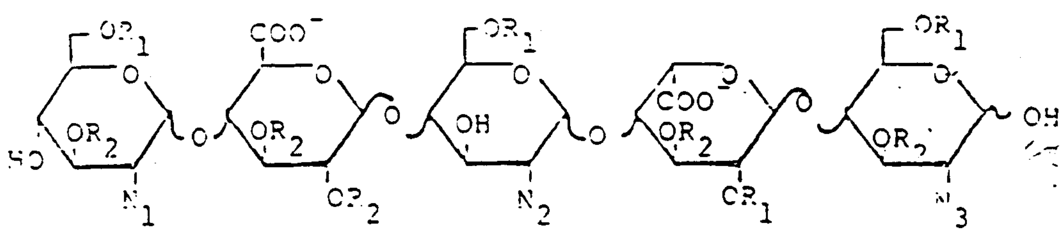

dans laquelle les substituants présentent les significations données ci-dessus et $N_3$ présente les significations données pour $N_1$ et $N_2$, de préférence de formule VI :

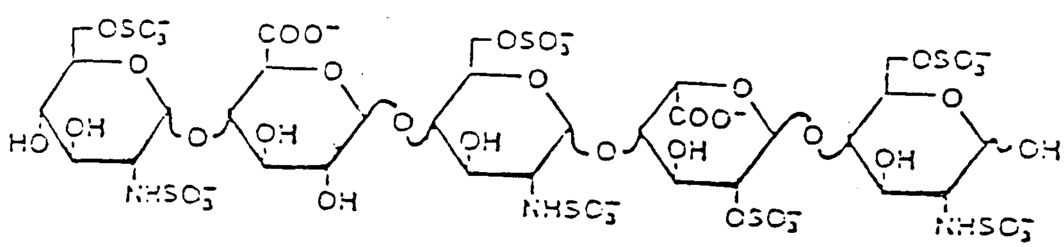

**10.** Les sels des oligosaccharides selon l'une quelconque des revendications 1 à 9, en particulier les sels de métaux, notamment, de métaux alcalins tels que le sodium, ou les sels de magnésium, de calcium ou d'une base organique azotée tel que du triéthylammonium.

**11.** Procédé de préparation de la structure DEFG de formule II selon la revendication 1, caractérisé en ce qu'il comprend :

- la réaction d'un halogénure, plus spécialement du bromure d'un disaccharide de structure EF, comportant un groupe temporaire en position 4 du motif E, avec un alcool d'un motif de structure G ;
- l'élimination du groupe temporaire de E pour recréer le groupe -OH ;
- la réaction de ce dernier avec l'halogénure plus spécialement le bromure du motif de structure D; la structure DEFGH de formule V étant plus spécialement préparée par :
- condensation d'un halogénure d'un motif E comprenant un groupe temporaire en position 4, et d'un alcool d'un motif F dont les positions 1 et 6 sont avantageusement bloquées par une fonction anhydro,
- introduction d'un halogénure, notamment, d'un bromure en position 1 du motif F,
- condensation avec un alcool de structure GH,
- élimination du groupe temporaire de E pour former un alcool, puis
- condensation avec un dérivé réactif d'un motif D, notamment un halogénure, en particulier, un bromure, avec élimination séquentielle des groupes protecteurs et introduction des substitutions spécifiques souhaitées, notamment en introduisant tout d'abord sélectivement les groupes sulfate après avoir éliminé les groupes -O-acétyle de blocage, cette réaction étant réalisée de manière à ne pas affecter les groupes benzyle et les groupes azotés et carboxyle présents, par exemple par saponification à l'aide d'une base forte telle que la soude, de préférence à une température inférieure à l'ambiante et plus spécialement voisine de 0 ° C, puis en soumettant le produit résultant de l'hydrolyse à l'action d'un agent d'alcoylation afin d'introduire, sur le groupe carboxyle, les groupes alcoyle protecteurs qui se sont trouvés éliminés lors de l'hydrolyse, et par réaction avec un agent de sulfatation, en introduisant des groupes sulfate aux positions libérées par l'hydrolyse et laissées libres après l'action de l'agent d'alcoylation, l'agent de sulfatation étant avantageusement un complexe triméthylamine/$SO_3^-$, cette réaction étant de préférence réalisée en milieu solvant, plus spécialement dans un solvant tel que le diméthylformamide, à une température supérieure à l'ambiante, généralement voisine de 50 ° C, l'introduction des groupes sulfate sur les fonctions alcool, étant alors suivie de la libération des groupes -OH bloqués par les radicaux protecteurs.

**12.** Médicaments caractérisés en ce qu'ils renferment une quantité efficace d'au moins un oligosaccharide selon l'une quelconque des revendications 1 à 10.

**13.** Médicaments caractérisés en ce qu'ils renferment une quantité efficace du tétrasaccharide de formule III.

**14.** Médicaments caractérisés en ce qu'ils renferment une quantité efficace du tétrasaccharide de formule IV.

## Claims

**1.** Oligosaccharides having the structure of fragments of acidic mucopolysaccharide chains or comprising such fragments, characterised in that they contain 4 to 12 components selected from amino sugar and uronic acid components, or the inverse thereof, and in that they contain a tetrasaccharide chain of the structure DEFG corresponding to formula II:

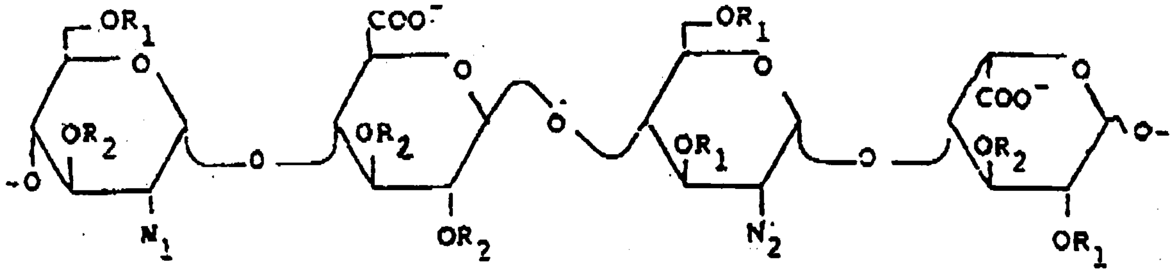

wherein:

the $R_1$ radicals, which may be identical or different from one another, represent a mineral anion esterifying the group -OH in position 6, end particularly a sulphate or phosphate group,

$R_2$ has one of the meanings given for $R_1$ or represents a hydrogen atom,

$N_1$ and $N_2$, which may be identical or different from one another, represent a functional amino group,

optionally substituted by an acyl group $-COR_3$ wherein $R_3$ represents an alkyl radical or a sulphamino or phosphamino group, and the salts thereof, with the exception of the pentasaccharide of formula:

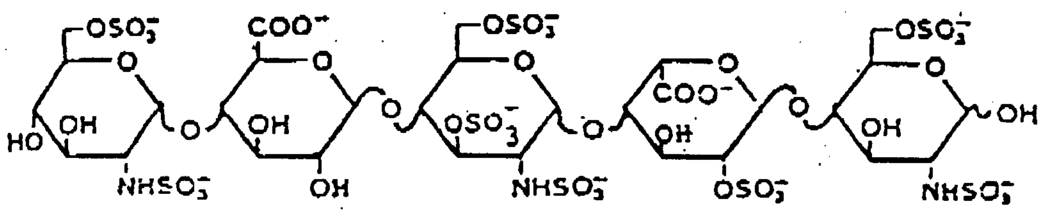

**2.** Oligosaccharides according to claim 1,

characterised in that they consist, apart from the DEFG sequence, or binary a-b D-glucuronic acid D-glucosamine, a-c L-iduronic acid D-glucosamine components, or the inverse thereof, of a single type of these binary chains, of several of these types of chains, or alternatively they contain one or more consecutive a, b or c components, or they contain one or more neutral sugar components and/or several deoxy sugars in their structure, these components being linked together by 1-2, 1-3, 1-4 or 1-6 bonds and, in the case of oligosaccharides having the structure of fragments of heparin or heparane sulphate, by c 1

$\xrightarrow{\alpha}$

4a, a 1

$\xrightarrow{\alpha}$

4b, a 1

$\xrightarrow{\alpha}$

4c and b 1

$\xrightarrow{\beta}$

4a bonds.

3. The DEFG tetrasaccharides of formula II per se.

4. Tetrasaccharides according to claim 3, characterised in that the substituent $R_2$ at position 3 of the component F represents a mineral anion esterifying the group -OH at position 3, and more especially a sulphate anion, at least some of the other substituents or more especially all the $R_1$ or $N_1$ and $N_2$ substituents also advantageously having a sulphate group.

5. Tetrasaccharide according to claim 4, characterised in that it corresponds to formula III:

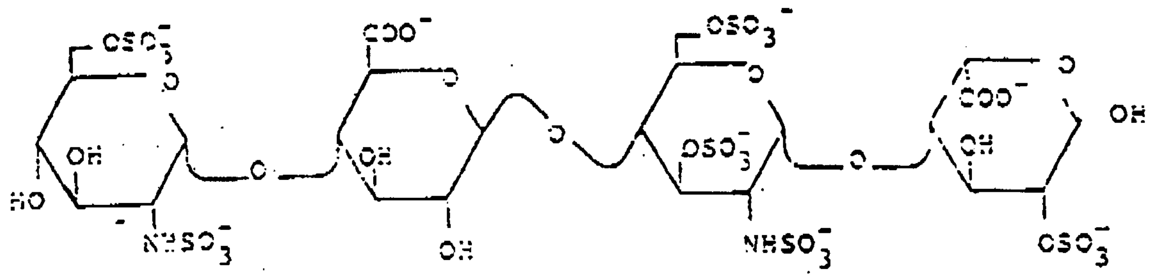

6. Tetrasaccharides according to claim 3, characterised in that the substituent $R_2$ at position 3 of component F denotes an -OH group, whilst at least some of the other substituents $R_1$ and/or $N_1$ and $N_2$ contain a sulphate group.

7. The oligosaccharides according to claim 1 containing a tetrasaccharide according to any of claims 4 to 6.

8. Oligosaccharides according to claim 7, characterised in that the DEFG sequence is followed by a D-glucosamine component.

9. Oligosaccharides according to claim 8, characterised in that they are pentasaccharides of structure DEFGH corresponding to formula V:

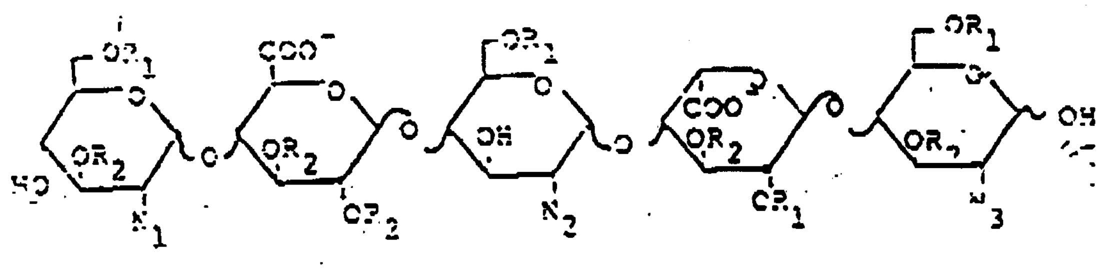

wherein the substituents are defined as hereinbefore and $N_3$ has the meanings given for $N_1$ and $N_2$, preferably of formula VI:

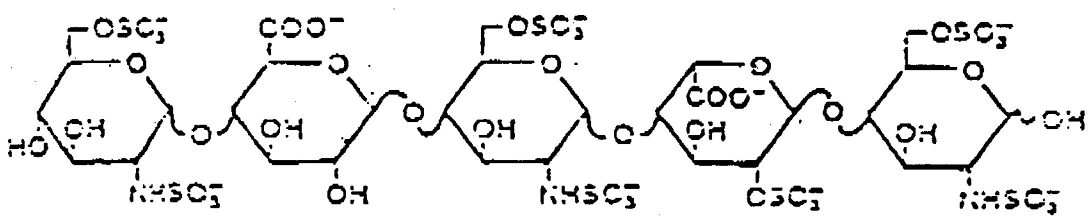

**10.** Salts of the oligosaccharides according to any one of claims 1 to 9, particularly metal salts and especially salts of alkali metals such as sodium, or salts of magnesium, calcium or a nitrogenated organic base such as triethylammonium.

**11.** Process for preparing the structure DEFG of formula II according to claim 1, characterised in that it comprises:

reacting a halide, more especially a bromide of a disaccharide of structure EF, having a temporary group at position 4 of the component E, with an alcohol of a component of structure G;

eliminating the temporary group from E in order to re-create the group -OH;

reacting the latter with the halide, more especially the bromide, of the component of structure D; the structure DEFGH of formula V being more especially prepared by:

condensation of a halide of a component E having a temporary group at position 4 and of an alcohol of a component F in which positions 1 and 6 are advantageously blocked by an anhydro function,

introduction of a halide, especially a bromide at position 1 of the component F,

condensation with an alcohol of structure GH,

elimination of the temporary group from E in order to form an alcohol, then

condensation with a reactive derivative of a component D, notably a halide and particularly a bromide, with sequential elimination of the protecting groups and introduction of the desired specific substituents, especially by first selectively introducing the sulphate groups after having eliminated the -O-acetyl blocking groups, this reaction being carried out in such a way as not to affect the benzyl groups and the nitro and carboxyl groups present, for example by saponification using a strong base such as sodium carbonate, preferably at a temperature below ambient temperature and especially it about 0°C, then subjecting the product obtained by hydrolysis to the action of an alkylating agent in order to introduce, at the carboxyl group, the protective alkyl groups which were eliminated during hydrolysis, and by reacting with a sulphating agent, by introducing sulphate groups at the positions freed by the hydrolysis and left free after the action of the alkylating agent, the sulphating agent advantageously being a trimethylamino/$SO_3^-$ complex, this reaction preferably being carried out in a solvent medium, more especially in a solvent such as dimethyl formamide, at a temperature above ambient temperature, generally around 50°C, the introduction of the sulphate groups into the alcohol functions being followed by the liberation of the -OH groups blocked by the protecting radicals.

**12.** Pharmaceutical compositions, characterised in that they contain on effective amount of at least one oligosaccharide according to any one of claims 1 to 10.

**13.** Pharmaceutical compositions, characterised in that they contain an effective amount of the tetrasaccharide of formula III.

**14.** Pharmaceutical compositions, characterised in that they contain an effective amount of the tetrasaccharide of formula IV.

**Patentansprüche**

**1.** Oligosaccharide, welche die Struktur von Fragmenten von sauren Mucopolysaccharidketten besitzen oder solche Fragmente enthalten,
dadurch **gekennzeichnet,** daß
sie 4 bis 12 Einheiten, die unter Aminozucker-Uronsäure-Einheiten oder umgekehrt aufgebauten Einheiten ausgewählt sind, und eine Tetrasaccharid-Sequenz der Struktur DEFG der Formel II

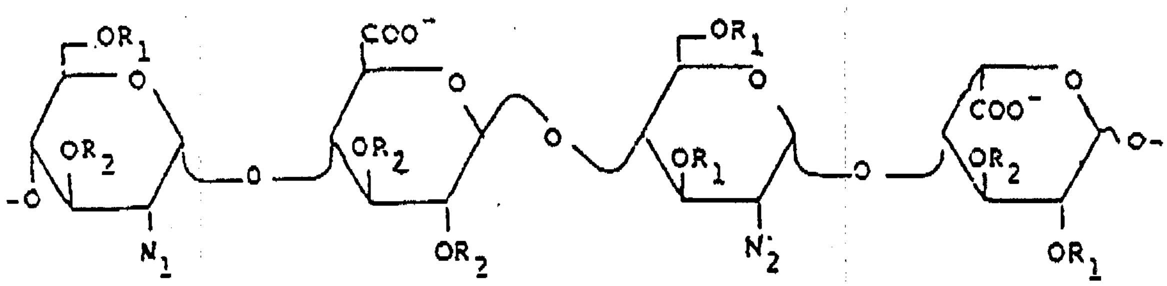

aufweisen, in der bedeuten:

- $R_1$, die gleich oder voneinander verschieden sein können, ein anorganisches Anion, mit dem die OH-Gruppe in 6-Stellung verestert ist, insbesondere eine Sulfatgruppe oder eine Phosphatgruppe,
- $R_2$ eine der Bedeutungen von $R_1$ oder ein Wasserstoffatom
  und
- $N_1$ und $N_2$, die gleich oder voneinander verschieden sein können, eine funktionelle Aminogruppe, die gegebenenfalls mit einer Acylgruppe $-COR_3$ substituiert ist, worin $R_3$ eine Alkylgruppe bedeutet, oder eine Sulfamino- oder Phosphoaminogruppe,
  sowie ihre Salze,
  wobei Pentasaccharide der Formel

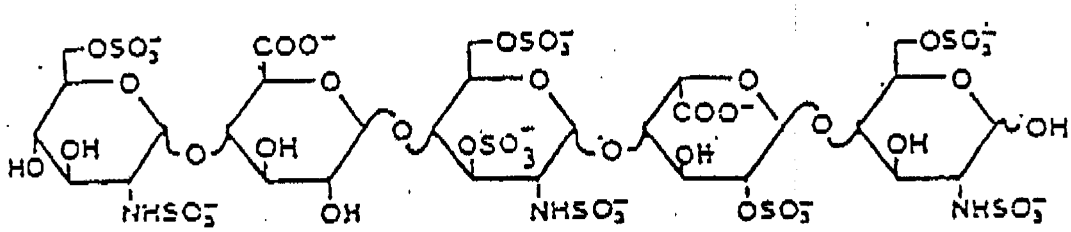

ausgenommen sind.

**2.** Oligosaccharide nach Anspruch 1,
dadurch gekennzeichnet, daß
sie, neben der Sequenz DEFG, aus binären Einheiten D-Glucosamin - D-Glucuronsäure, a-b, D-Glucosamin - L-Iduronsäure, a-c, oder umgekehrt aufgebauten Einheiten, aus einer einzigen Art dieser binären Gruppierungen oder aus mehreren dieser Gruppierungen aufgebaut sind oder gemäß einer Variante eine oder mehrere aufeinanderfolgende Einheiten a, b oder c aufweisen oder ferner eine oder mehrere Einheiten von neutralen Zuckern und/oder mehrere Desoxyzucker in ihrer Struktur enthalten, wobei diese Einheiten durch 1,2-, 1,3-, 1,4- oder 1,6-Verknüpfungen und im Fall der Oligosaccharide, welche die Struktur von Fragmenten von Heparin oder Heparansulfat besitzen, durch c 1

$\xrightarrow{\alpha}$

4 a-, a 1

$\xrightarrow{\alpha}$

4 b-, a 1

$\xrightarrow{\alpha}$

4 c- und b 1

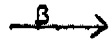

4a-Verknüpfungen untereinander verbunden sind.

**3.** Die DEFG-Tetrasaccharide der Formel II als solche.

**4.** Tetrasaccharide nach Anspruch 3,
dadurch gekennzeichnet, daß
der Substituent $R_2$ in 3-Stellung der Einheit F ein anorganisches Anion darstellt, das die OH-Gruppe in 3-Stellung verestert, insbesondere ein Sulfat-Anion, wobei mindestens einige der übrigen Substituenten oder insbesondere sämtliche Substituenten $R_1$ oder $N_1$ und $N_2$ ebenfalls vorteilhaft eine Sulfatgruppe tragen.

**5.** Tetrasaccharid nach Anspruch 4,
dadurch gekennzeichnet, daß
es der Formel III entspricht:

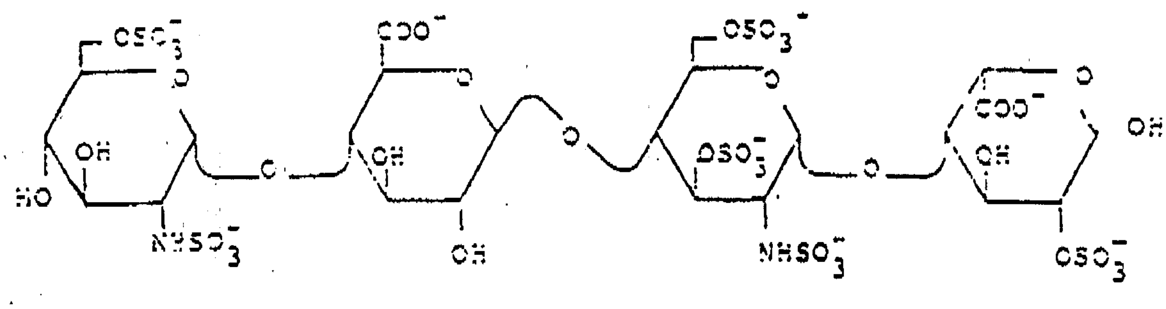

**6.** Tetrasaccharide nach Anspruch 3,
dadurch gekennzeichnet, daß
der Substituent $R_2$ in 3-Stellung der Einheit F eine OH-Gruppe darstellt, wobei mindestens einige der übrigen Substituenten $R_1$ und/oder $N_1$ und $N_2$ eine Sulfatgruppe tragen.

**7.** Oligosaccharide nach Anspruch 1,
die ein Tetrasaccharid nach einem der Ansprüche 4 bis 6 enthalten.

**8.** Oligosaccharide nach Anspruch 7,
dadurch gekennzeichnet, daß
sich an die Sequenz DEFG eine D-Glucosamin-Einheit anschließt.

**9.** Oligosaccharide nach Anspruch 8,
dadurch gekennzeichnet, daß
es sich um Pentasaccharide der Struktur DEFGH der Formel V handelt,

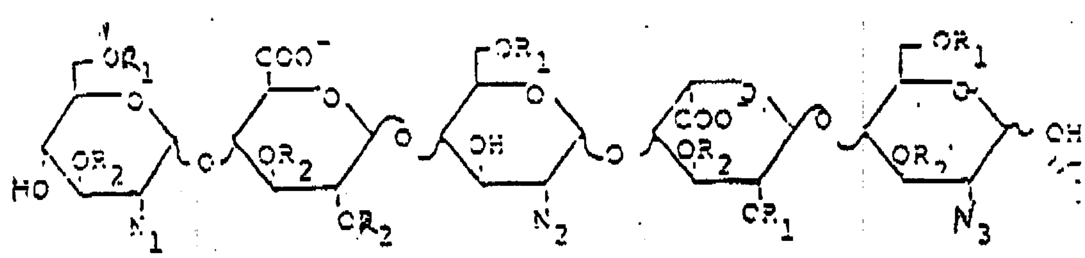

in der die Substituenten die oben angegebenen Bedeutungen besitzen und $N_3$ die für $N_1$ und $N_2$ angegebenenBedeutungen besitzt, vorzugsweise der Formel VI:

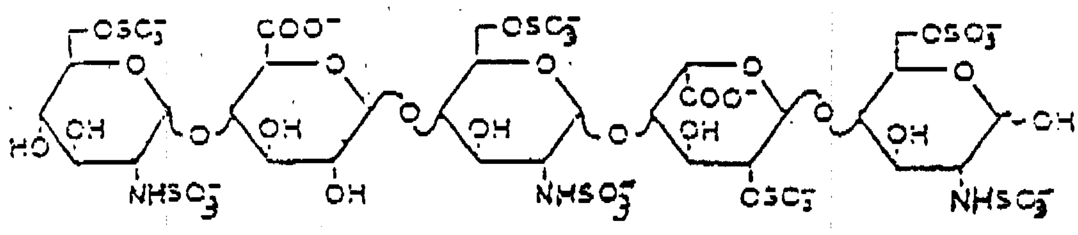

10. Salze der Oligosaccharide nach einem der Ansprüche 1 bis 9, insbesondere die Metallsalze und besonders die Alkalimetallsalze, wie die Natriumsalze, oder die Magnesium- oder Calciumsalze oder die Salze mit einer organischen Stickstoffbase, wie die Triethylammoniumsalze.

11. Verfahren zur Herstellung der Sequenz DEFG der Formel I nach Anspruch 1,
dadurch gekennzeichnet, daß es folgende Schritte umfaßt:
- Umsetzung eines Halogenids, insbesondere des Bromids, eines Disaccharids der Struktur EF, das eine temporäre Gruppe in 4-Stellung der Einheit E aufweist, mit einem Alkohol einer Einheit der Struktur G;
- Eliminierung der temporären Gruppe der Einheit E zur Freisetzung der OH-Gruppe;
- Umsetzung der letzteren mit dem Halogenid, insbesondere dem Bromid, der Einheit der Struktur D,

wobei die Sequenz DEFGH der Formel V insbesondere hergestellt wird durch:
- Kondensation eines Halogenids einer Einheit E, die eine temporäre Gruppe in 4-Stellung aufweist, mit einem Alkohol einer Einheit F, bei der die 1-Stellung und die 6-Stellung vorteilhaft mit einer Anhydrofunktion blockiert sind,
- Einführung eines Halogenids, insbesondere eines Bromid in 1-Stellung der Einheit F,
- Kondensation mit einem Alkohol der Struktur GH,
- Eliminierung der temporären Gruppe von E unter Erzeugung eines entsprechenden Alkohols und anschließend
- Kondensation mit einem reaktiven Derivat einer Einheit D, insbesondere einem Halogenid, und besonders einem Bromid, unter sequentieller Eliminierung der Schutzgruppen und Einführung der speziellen gewünschten Substituenten, insbesondere so, daß zunächst selektiv die Sulfatgruppen eingeführt werden, nachdem die zur Blockierung verwendeten O-Acetylgruppen abgespalten wurden, wobei diese Umsetzung derart durchgeführt wird, daß die Vorliegenden Benzylgruppen, stickstoffhaltigen Gruppen und Carboxylgruppen nicht abgespalten werden, beispielsweise durch Verseifung mit einer starken Base, wie Natriumhydroxid, vorzugsweise bei einer Temperatur unter Raumtemperatur und insbesondere in der Nähe von 0 °C, worauf das von der Hydrolyse resultierende Produkt mit einem Alkylierungsmittel umgesetzt wird, um an den Carboxylgruppen als Schutzgruppen dienende Alkylgruppen einzuführen, die bei der Hydrolyse abgespalten wurden, und anschließend mit einem Sulfatierungsmittel umgesetzt wird, wobei in den durch Hydrolyse freigesetzten Stellungen und den nach Einwirkung des Alkylierungsmittels frei gebliebenen Stellungen Sulfatgruppen eingeführt werden und das Sulfatierungsmittel vorteilhaft ein Trimethylamin/$SO_3^-$ -Komplex ist, wobei diese Umsetzung vorzugsweise in einem Lösungsmittelmedium und insbesondere einem Lösungsmittel wie Dimethylformamid bei einer über Raumtemperatur liegenden Temperatur, allgemein um 50 °C, durchgeführt wird, worauf sich an die Einführung der Sulfatgruppen in den Alkoholfunktionen dann die Freisetzung der durch Schutzgruppen blockierten OH-Gruppen anschließt.

12. Arzneimittel,
dadurch gekennzeichnet, daß
sie eine wirksame Menge mindestens einen Oligosaccharids nach einem der Ansprüche 1 bis 10 enthalten.

13. Arzneimittel,
dadurch gekennzeichnet, daß
sie eine wirksame Menge des Tetrasaccharids der Formel III enthalten.

**14.** Arzneimittel,
dadurch gekennzeichnet, daß
sie eine wirksame Menge des Tetrasaccharids der Formel IV enthalten.

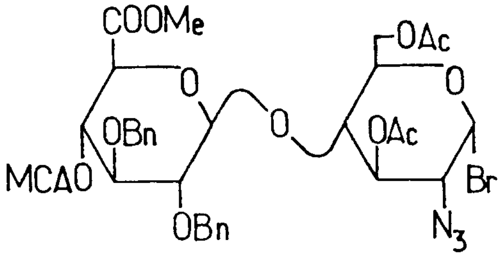

+

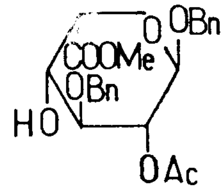

1 2

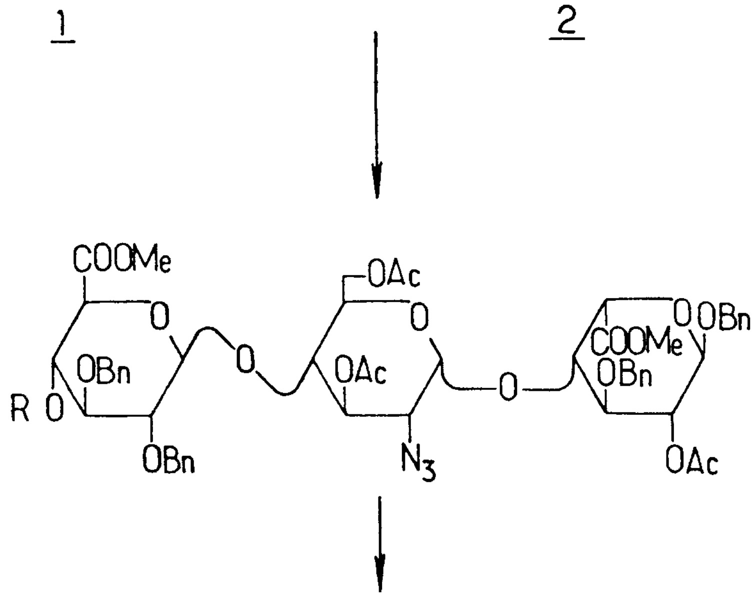

3 R=MCA

4 R=H

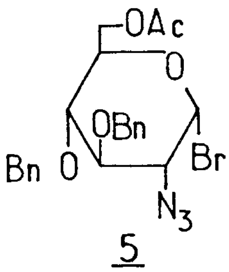

+ 4

5

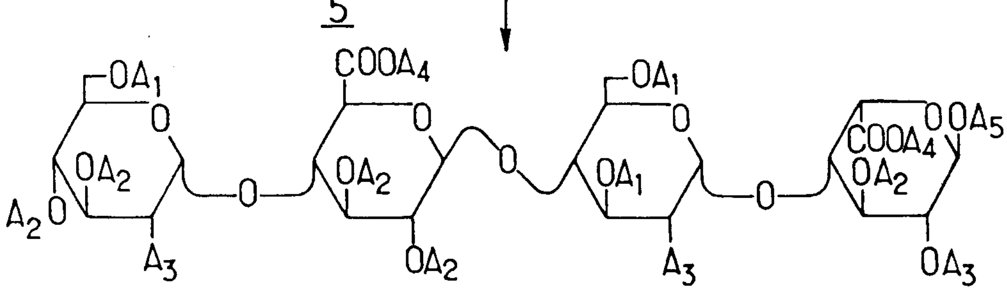

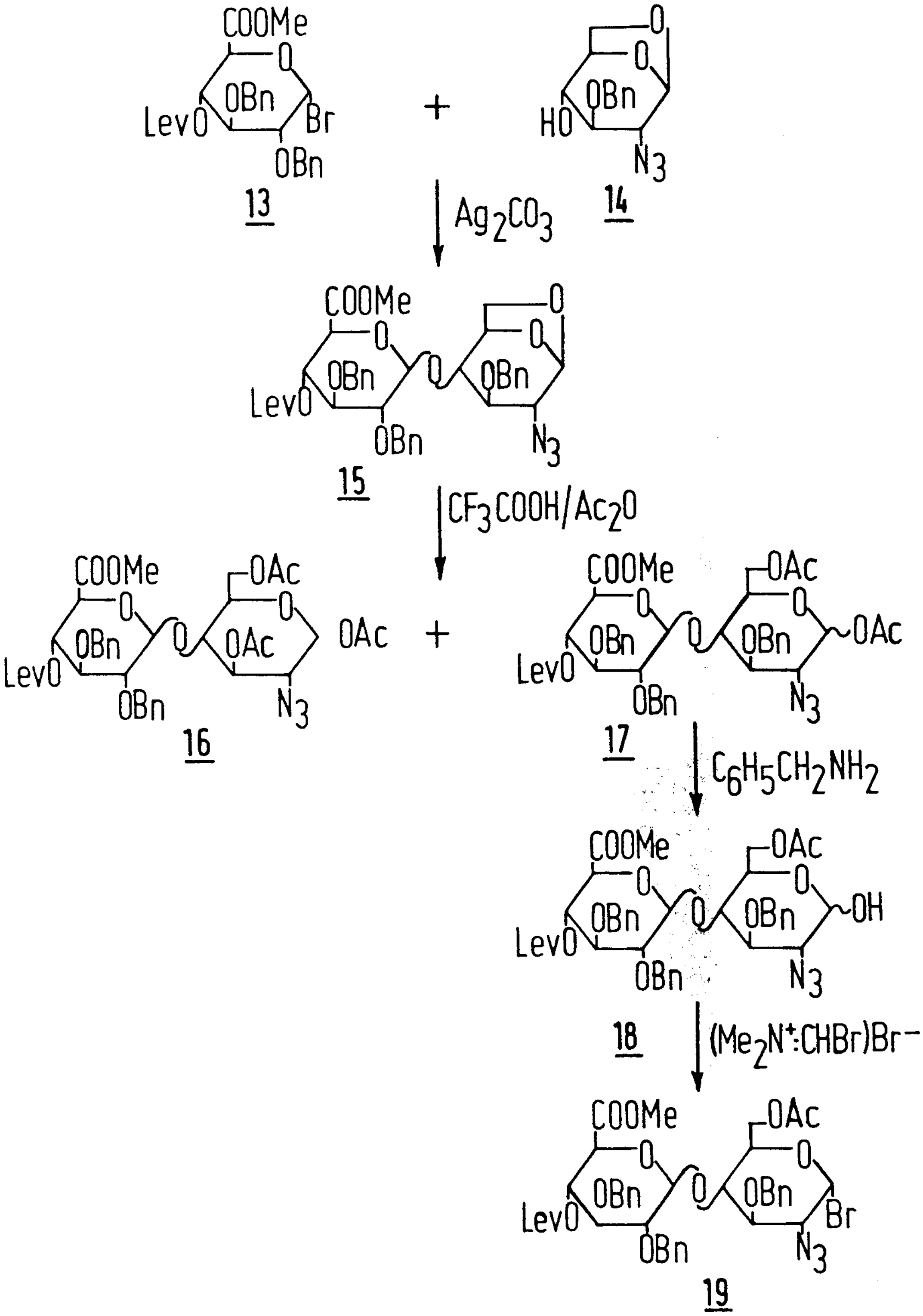


13 14 15 16 17 18 19

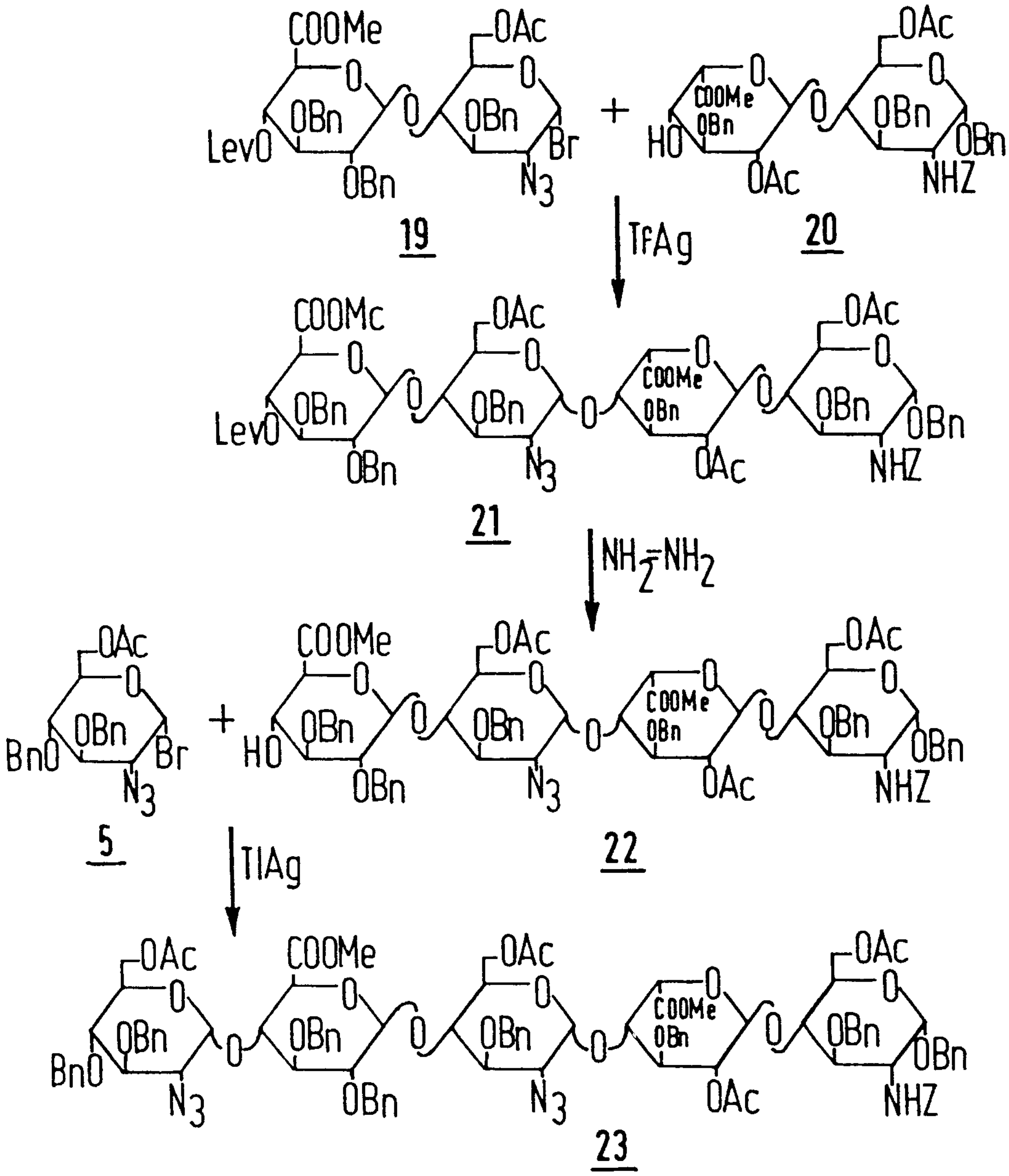


19 20

21

5 22

23

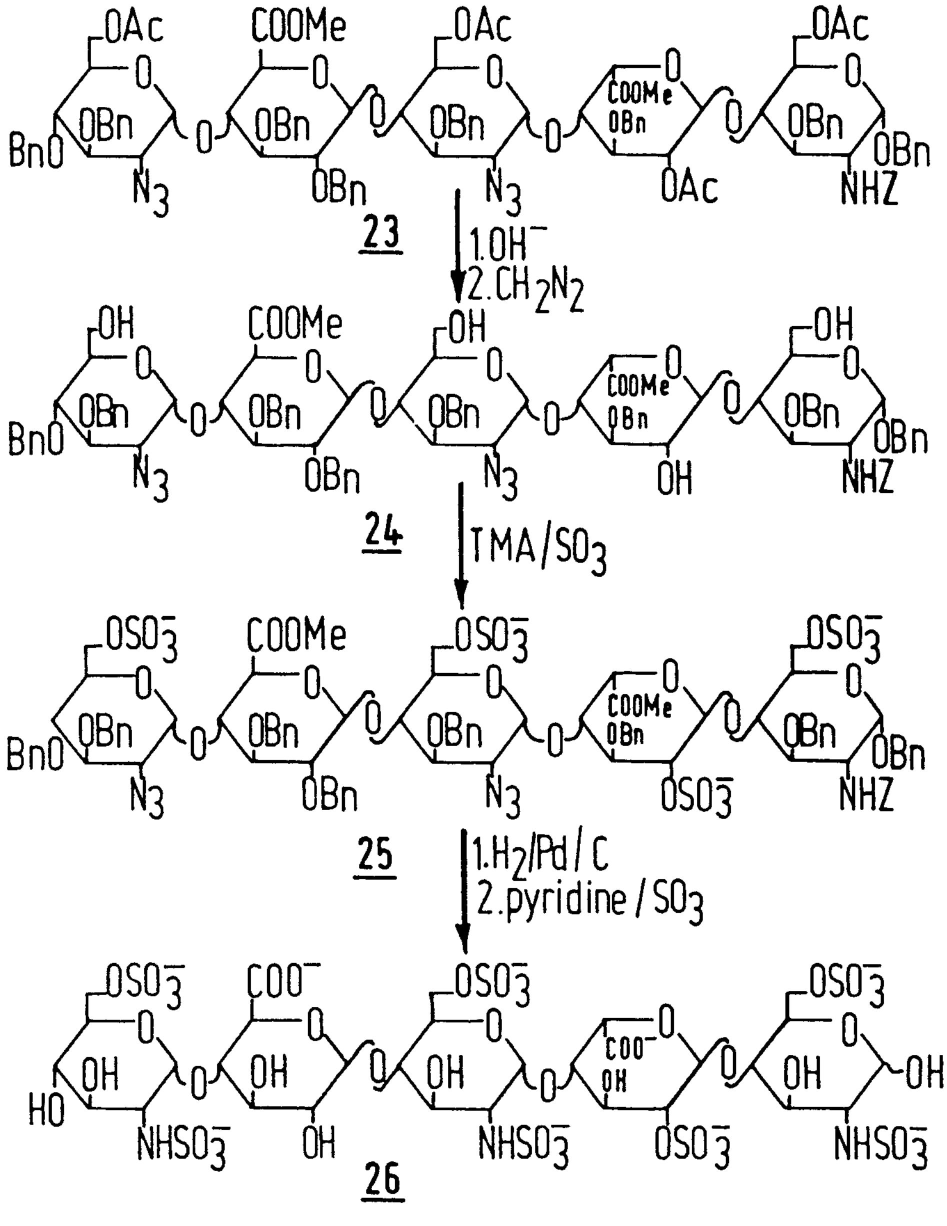


23

24

25

26

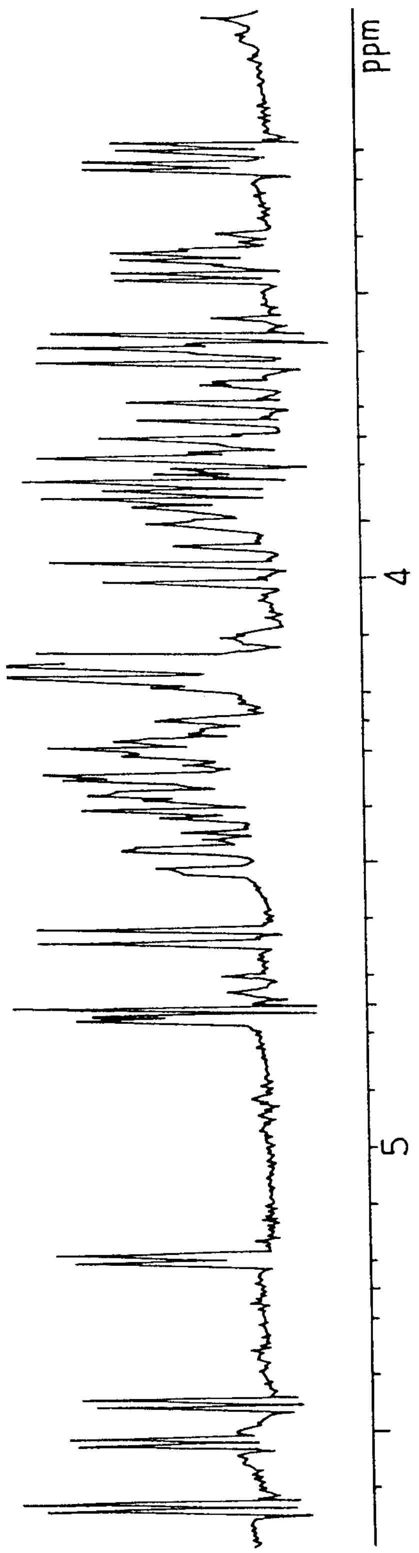
ppm
4
5

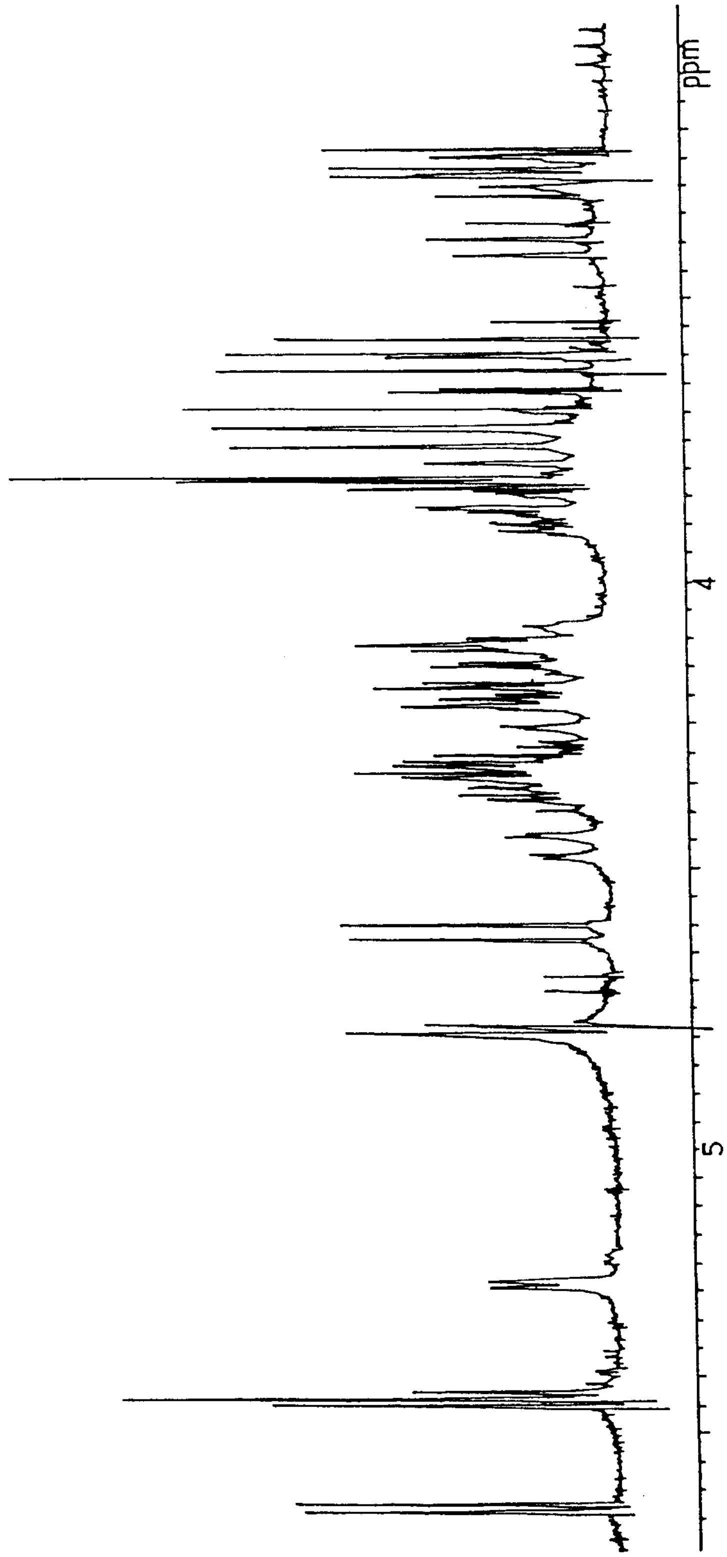
ppm
4
5